(19) Europäisches Patentamt
European Patent Office
Office européen des brevets

(11) EP 3 945 088 A1

(12) # EUROPÄISCHE PATENTANMELDUNG

(43) Veröffentlichungstag:
**02.02.2022 Patentblatt 2022/05**

(21) Anmeldenummer: **20188528.2**

(22) Anmeldetag: **30.07.2020**

(51) Internationale Patentklassifikation (IPC):
***C07C 67/39*** *(2006.01)* ***C07C 69/54*** *(2006.01)*

(52) Gemeinsame Patentklassifikation (CPC):
(C-Sets verfügbar)
**C07C 67/39** (Forts.)

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(71) Anmelder: **Röhm GmbH**
**64295 Darmstadt (DE)**

(72) Erfinder: **Die Erfindernennung liegt noch nicht vor**

(74) Vertreter: **Röhm Patent Association**
**IP Management**
**Deutsche-Telekom-Allee 9**
**64295 Darmstadt (DE)**

(54) **VORGEHEN ZUR MINIMIERUNG DES AKTIVITÄTSVERLUSTS BEI IM KREISLAUFBETRIEB AUSGEFÜHRTEN REAKTIONSSCHRITTEN**

(57) Die vorliegende Erfindung betrifft ein neuartiges Verfahren zur Herstellung von Methacrylaten, wie Methacrylsäure und/oder Alkylmethacrylaten, insbesondere MMA. Dabei betrifft die vorliegende Erfindung eine Verlängerung der Katalysatorstandzeit und Effizienzsteigerung von auf C2- oder C4-Rohstoffen basierten Herstellungsverfahren, also insbesondere solchen Verfahren, die von Isobutylen oder tert-Butanol oder Ethylen als Rohstoff ausgehen.

Es ist mit dem vorliegenden erfindungsgemäßen Verfahren gelungen, solche Verfahren bei gleichbleibenden oder sogar gesteigerten Aktivitäten und Selektivitäten länger störungsfrei zu betreiben. Hieraus ergibt sich die Möglichkeit, solche Verfahren möglichst einfach, wirtschaftlich und umweltschonend durchzuführen. Zusätzlich ist es mit dem vorliegenden erfindungsgemäßen Verfahren gelungen, bekannte, vom Zwischenprodukt Methacrolein ausgehende Sicherheitsrisiken zu minimieren.

EP 3 945 088 A1

(52) Gemeinsame Patentklassifikation (CPC): (Forts.)

C-Sets
**C07C 67/39, C07C 69/54**

## Beschreibung

### Gebiet der Erfindung

Die vorliegende Erfindung betrifft eine Vorgehensweise zur Minimierung des Aktivitätsverlusts bei im Kreislaufbetrieb ausgeführten Reaktionsschritten. Bei zahlreichen Verfahren der chemischen Industrie wird ein solcher Kreislaufbetrieb angewendet, wenn in einem Reaktionsschritt nur ein Teil des dem Reaktor zugeführten Rohstoffs in das Zielprodukt umgewandelt wird und der verbleibende Rest des Rohstoffs nach dem Reaktor wieder zum Eingang des Reaktors zurückgeführt wird. Ziel einer solchen partiellen Umsetzung kann es beispielsweise sein, die irreversible Bildung von Nebenprodukten zu minimieren, die vermehrt im Bereich hoher Umsätze sowie, bei exothermen Reaktionen, durch die bei erhöhten Umsätzen auftretenden erhöhten Reaktionstemperaturen entstehen. Die Bildung solcher Nebenprodukte erhöht die spezifischen Rohstoffkosten des gewünschten Produkts und zieht Kosten für die Entsorgung der Nebenprodukte nach sich.

### Stand der Technik

Durch die Kreislauffahrweise kann erreicht werden, dass das Zielprodukt in hoher Ausbeute aus dem Rohstoff gewonnen werden kann, ohne durch die typischerweise im hohen Umsatzbereich auftretenden Nebenprodukte Verluste zu erleiden.

Im Allgemeinen wird hierfür der im Reaktionsschritt nicht umgesetzte Rohstoff vom Zielprodukt durch einen geeigneten Verfahrensschritt abgetrennt und, gegebenenfalls zusammen mit anderen ebenfalls vom Zielprodukt abgetrennten Komponenten, dem Eingangsstrom des Reaktors wieder zugeführt. Solche Verfahrensschritte können umfassen, sind jedoch nicht beschränkt auf, Extraktionen, Kristallisationen, Destillationen, Kondensationen oder Membrantrennverfahren. Ein allgemeines Prozessschema ist in Abbildung 1 aufgeführt.

Der derart abgetrennte, nicht umgesetzte Rohstoff wird häufig in einer geeigneten Vorrichtung zwischengelagert, bevor er dem Eingangsstrom des Reaktors wieder zugeführt wird. Solche Vorrichtungen umfassen, sind jedoch nicht beschränkt auf, Gasometer, Druckbehälter oder Lagertanks. Vorteil einer solchen Speicherung ist, dass die Konzentration des nicht umgesetzten Rohstoffs im Eingangsstrom des Reaktors leicht konstant gehalten werden kann, indem ggf. infolge veränderlicher Umsatzgrade schwankende Massenströme des nicht umgesetzten Rohstoffs ausgeglichen werden. Dies ist insbesondere dann von Vorteil, wenn die nicht umgesetzten Rohstoffströme mehrerer gleichartig und parallel ausgeführter Reaktionsschritte in einer gemeinsamen Vorrichtung gesammelt und allen Reaktoren in gleicher Verteilung wieder zugeführt werden sollen. Eine Verstetigung der Eingangskonzentration an nicht umgesetztem Rohstoff vermeidet die Anpassung von Betriebsparametern wie beispielsweise Druck und Temperatur im Reaktionsschritt und gewährleistet so einen sicheren und störungsfreien Betrieb der Produktion.

Eine solche Vorrichtung zur Sammlung wird üblicherweise so ausgeführt, dass die Zeit bis zur notwendigen Anpassung der Betriebsparameter möglichst lang ist. Idealerweise wird beispielsweise ein Zwischenlagertank mit einem großen Volumen ausgeführt, so dass eine stetige Rückführung von nicht umgesetztem Rohstoff auch über einen langen Zeitraum möglich ist. Zudem ist es vorteilhaft, wenn Parameter wie Druck und Temperatur während der Zwischenlagerung möglichst nah an den Parametern des Eingangsstroms zum Reaktionsschritt gewählt sind. Damit können energieintensive Kompressions-, Heiz- oder Kühlschritte vermieden werden. In diesem Sinne ist beispielsweise die Zwischenlagerung nicht umgesetzter Rohstoffe bei einer der Eingangstemperatur des Reaktionsschritts ähnlichen Temperatur energetisch sehr günstig.

Beispiele solcher im Kreislaufbetrieb ausgeführten Reaktionsschritte umfassen, sind jedoch nicht beschränkt auf, die partielle Flüssigphasen-Hydrierung, die partielle GasphasenOxidation oder die oxidative Veresterung von ungesättigten Carbonylverbindungen. Typischerweise werden solche Reaktionen durch Katalysatoren, welche hier auch als "Kontakte" bezeichnet werden können, beschleunigt. Dabei können diese beispielsweise auch Teil einer mehrstufigen Reaktionskaskade, bei der das Zielprodukt eines ersten Reaktionsschritts der Rohstoff eines zweiten Reaktionsschritts ist, sein. Weiterhin kann auch der zweite Reaktionsschritt im Kreislaufbetrieb ausgeführt sein, sowie das Zielprodukt des ersten Reaktionsschritts kann ebenfalls in einer dazu geeigneten Vorrichtung des zweiten Reaktionsschritts als Rohstoff zwischengelagert werden.

Ein wichtiges Beispiel einer solchen mehrstufigen Reaktionskaskade ist ein Verfahren zur zweistufigen Oxidation von C4-Grundkörpern wie Isobuten (IBEN) oder tert-Butanol (TBA) in Gegenwart von Wasser und Luft zur Herstellung von Methacrylsäure über die Zwischenstufe Methacrolein.

Die ungesättigte Carbonsäure Methacrylsäure (MAS) wird als Ausgangsstoff zur Herstellung von Kunststoffen verwendet. Die Salze und Ester der Methacrylsäure werden als Methacrylate bezeichnet. Unter diesen ist ein bekannter und bedeutender Vertreter der Methacrylsäuremethylester, bzw. Methylmethacrylat (MMA).

Jährlich werden mehr als 3 Millionen Tonnen MAS produziert, die zum Großteil als Ausgangsstoff für die Synthese anderer chemischer Verbindungen dienen. Industriell kann MAS ausgehend von den Steamcracker-Produkten

Ethen (C2-Grundkörper) oder Isobuten (C4-Grundkörper) über die Zwischenstufe Methacrolein (MAL) gewonnen werden.

MAL selbst wird als Zwischenprodukt zur Herstellung von Polymeren, insbesondere z. B. Polymethylmethacrylat [PMMA], Harzen, Pflanzenschutzmitteln und in geringem Maße für Geschmacks- und Geruchsstoffe verwendet.

Bei der Herstellung von Methacrylaten aus dem C2-Grundkörper Ethylen wird aus Ethylen durch Hydroformylierung gewonnener Propionaldehyd zunächst in einem ersten Reaktionsschritt mit Formaldehyd zu MAL umgesetzt. In einem zweiten Reaktionsschritt kann dieses MAL anschließend mit Methanol katalytisch zu MMA oxidativ verestert werden. Ein entsprechendes Verfahren ist in der WO 2014/170223 beschrieben. Alternativ kann auch dieses MAL zu MAS oxidiert und optional anschließend mit Methanol zu MMA verestert werden.

Bei der Herstellung von MMA aus C4-Grundkörpern werden im allgemeinen IBEN oder TBA oder Mischungen aus IBEN und TBA zunächst in einer ersten Reaktionsstufe in der Gasphase zusammen mit einem Sauerstoff enthaltenden Gasgemisch an einem heterogenen Katalysator ("Kontakt 1") zu einem MAL-haltigen Gasgemisch umgesetzt. Der Umsatz des C4-Grundkörpers ist hierbei nahezu quantitativ und wird beispielsweise mit größer 99% angegeben. Verfahren zur Oxidation von C4-Grundkörpern zu MAL sind beispielsweise in der DE 10 2006 015710 oder der EP 19 952 32 beschrieben. Die aus der ersten Reaktionsstufe austretende Prozessgasphase wird mit einer kühleren, recyclierten MAL enthaltenden Gasphase zusammen mit Luftsauerstoff und Wasserdampf vermischt. Somit ergibt sich das Feedgas der zweiten Stufe.

Ein solchermaßen hergestelltes MAL-haltiges Gasgemisch wird anschließend in einer zweiten Reaktionsstufe in der Gasphase an einem weiteren heterogenen Katalysator ("Kontakt 2") zu einem MAS-haltigen Gasgemisch umgesetzt.

Die zweite Oxidationsstufe wird wie die erste bei einem moderaten Überdruck zwischen 0.1 und 2 bar und bei Temperaturen zwischen 260 und 360 °C betrieben. Hierzu kommen Heteropolysäurekontakte auf Basis von Molybdän und Phosphor sowie einigen weiteren Dopanten zum Einsatz (siehe z.B. US2007/0010394). Die modifizierten Heteropolysäuren erreichen bei höheren Umsätzen tendenziell signifikant schlechtere Selektivitäten. Aus diesem Grund wird der Umsatz und die damit verbundene Katalysatorbelastung zwischen 65 % und 85 % eingestellt. Aus dieser Tatsache ergibt sich für alle Verfahren und deren Modifikationen die Notwendigkeit, nicht umgesetztes Methacrolein aus dem Prozessgas vom gewünschten Produkt Methacrylsäure abzutrennen und letztlich als sogenanntes Recycle-MAL vor den zweiten Oxidationsreaktor zurückzufahren.

Eine solche Abtrennung von Methacrolein wird üblicherweise durch mindestens einen Destillations- oder Extraktionsschritt durchgeführt. MAL selbst wird aus dem Prozessgas absorbiert und weiterverarbeitet, teilweise gereinigt, durch ein Desorptionsverfahren und gegebenenfalls mindestens einen Destillationsvorgang isoliert. Nach der Absorption und Desorption erfolgt gegebenenfalls eine weitere Destillation. Durch das Vorhandensein leichtflüchtiger, aber kondensierbarer Komponenten zusätzlich zum Acrolein erhält man je nach Trennaufwand Roh-Methacrolein mit einigen Bestandteilen an organischen Nebenprodukten, meist ein MAL-Gemisch mit einer MAL-Konzentration größer 70 Gew%. So wird z.B. in der GB 2004886 das Durchleiten einer MAL-haltigen Reaktionsmischung durch eine Quench-Kolonne, in der hochsiedende Komponenten wie Methacrylsäure kondensiert werden, beschrieben. Das im Rest-Gas verbleibende MAL wird anschließend absorbiert und nach Strippen in gasförmiger Form der zweiten Oxidationsstufe zugeführt.

Das von der Methacrylsäure getrennte Methacrolein-haltige Gemisch nach der zweiten Reaktionsstufe enthält je nach Katalysatorgüte und Parametern der Verfahrensdurchführung neben Methacrolein noch weitere Nebenprodukte wie unter anderem Aldehyde. Als Stand der Technik kann für das Recycle-MAL folgendes Nebenproduktspektrum eingegrenzt werden:

| | |
|---|---|
| 0,5 - 4 Gew% | Acetaldehyd |
| 1 - 8 Gew% | Aceton |
| 1 - 5 Gew% | Acrolein |
| 0,05 - 0,4 Gew% | Butan-2,3-dion |
| 0,2 - 1,5 Gew% | MMA |
| 1 - 5 Gew% | Wasser |
| 1 - 5 Gew% | Methacrylsäure |
| 0,1 - 3 Gew% | Essigsäure |
| Und damit | |
| 70 - 95 Gew% | Methacrolein |

Kennzeichnend für das Recycle-MAL ist je nach Verfahren, wie zum Beispiel in einem Tandem-Betrieb der ersten und zweiten Oxidationsstufe oder Zwischenisolierung von MAL, ein Methacroleingehalt größer 70 Gew% und die Anwesenheit sowohl leichter siedenden Komponenten wie Aceton, Acrolein und Acetaldehyd, als auch höher siedende Komponenten wie MMA, Wasser und Methacrylsäure.

Die nach der zweiten Reaktionsstufe erhaltene MAS wird nachfolgend mit Methanol in MMA überführt. Übersichtliche Zusammenfassungen zum C4-basierten Verfahren sind unter anderem in Ullmann's Encyclopedia of Industrial Chemistry 2012, Wiley-VCH Verlag GmbH & Co. KGaA, Weinheim, Methacrylic Acid and Derivatives, DOI: 10.1002/14356007.a16_441.pub2 sowie in Krill und Rühling et. al. "Viele Wege führen zum Methacrylsäuremethylester", WILEY-VCH Verlag GmbH & Co. KGaA, Weinheim, doi.org/10.1002/ciuz.201900869 dargelegt.
Insgesamt sind für die C4-basierte MMA-Herstellung folgenden drei Wege gut bekannt:

Verfahren A, "Tandem C4-Direkt-Oxidationsverfahren (Sumitomo-Verfahren)", ohne Zwischenisolierung von Methacrolein: Hier wird in einem ersten Schritt MAL aus Isobuten hergestellt, das in einem Verfahrensschritt 2 zu MAS oxidiert wird, bevor in einem Verfahrensschritt 3 durch Veresterung von MAS mit Methanol MMA erhalten wird. Dieses Verfahren wird in der Literatur auch als "Tandemverfahren" bezeichnet, da das Prozessgas der ersten Stufe ohne Isolierung des Zwischenproduktes MAL direkt zu MAS oxidiert wird.

Verfahren B, "Getrennte C4-Direktoxidation (Mitsubishi-Verfahren)": Hier wird, ähnlich wie bei Verfahren A, in einem ersten Verfahrensschritt MAL aus Isobuten hergestellt, das in einem separaten Verfahrensschritt in flüssiger Form isoliert und gereinigt wird, bevor es in einem Verfahrensschritt 3 verdampft und zu MAS oxidiert und schließlich in einem Verfahrensschritt 4 durch Veresterung zu MMA umgewandelt wird.

Verfahren C, "Direktes Metha-Verfahren (Asahi-Verfahren)" oder direktes oxidatives Veresterungsverfahren: Auch hier wird in einem ersten Verfahrensschritt MAL in der Gasphase an einem ersten Kontakt aus Isobuten hergestellt, das ebenfalls zunächst in einem Verfahrensschritt 2 isoliert und zwischengereinigt wird, bevor es in einem Verfahrensschritt 3 direkt oxidativ zu MMA verestert wird. Verfahrensschritt 3 wird in der flüssigen Phase an einem suspendierten Kontakt durchgeführt. Somit ist die Kombination aus einem Gasphasenschritt und einem Prozessschritt in der Flüssigphase der wesentliche Unterschied dieses Verfahrens im Vergleich zu den Verfahren A und B, bei denen beide Partialoxidationen an unterschiedlichen Kontakten jeweils in der Gasphase durchgeführt werden.

Alle beschriebenen Prozesse gemäß Stand der Technik sind gut dokumentiert, so z.B. in (i) IHS Chemical Process Economics Program, Review 2014-05, R.J. Chang, Syed Naqvi oder (ii) S. Nakamura, H. Ichihashi, Vapor Phase Catalytic Oxidation of Isobutene to Methacrylic Acid, Stud. Surf. Sci. Catal. 1981, 7, 755-767.

Die Partialoxidation von MAL zu MAS gemäß Verfahren A oder B verläuft exotherm und kann durch den Einsatz von Heteropolysäure- (HPA-) Katalysatoren als "Kontakt 2" beschleunigt werden. Hierfür wird ein Gasgemisch - auch als "Feed" bezeichnet - bestehend aus Luft, Wasserdampf, nicht umgesetztem, zurückgeführten Reaktionsgas und nicht umgesetztem, zurückgeführtem MAL am Kontakt 2 oxidiert. Der hierfür verwendete Reaktor wird üblicherweise als Rohrbündelapparat ausgeführt und die dabei freiwerdende Wärme über ein in einer oder mehreren Zonen ausgeführtes Salzbad abgeführt. Wie in der EP 0006248 beschrieben, kann der Kontakt 2 Formulierungen umfassen, die Molybdän, Vanadium, Phosphor und Sauerstoff beinhalten.

Während die Oxidation von MAL zu MAS in der zweiten Reaktionsstufe mit einem frischen Kontakt schon bei vergleichsweise niedrigen Salzbadtemperaturen von beispielsweise T = 260 bis 300 °C durchgeführt werden kann, muss die Salzbadtemperatur im Verlauf der Lebenszeit des Katalysators, z.B. auf 310 bis 330 °C erhöht werden, um einen Alterungsbedingten Aktivitätsverlust des Katalysators auszugleichen. Durch die Erhöhung der Salzbadtemperatur werden der Umsatz und die auf den einfachen Durchgang durch das Katalysatorbett bezogene MAS-Ausbeute konstant gehalten, was Voraussetzung für den Betrieb des Verfahrens in einem wirtschaftlich sinnvollen Bereich ist.

Durch die Erhöhung der Salzbadtemperatur steigt auch die Temperatur des Reaktionsgases kontinuierlich an. Gegen Ende der Lebensdauer des Katalysators, die je nach Gasbelastung, ausgedrückt als GHSV ("gas hourly space velocity"), üblicherweise bei 1 bis 3 Jahren liegt, erreicht die Temperatur des Reaktionsgases einen kritischen Bereich, in dem es zu einer autokatalytischen Reaktion innerhalb des Sauerstoff- und Methacrolein-haltigen Reaktionsgemischs, dem sogenannten "Post Combustion"-Phänomen, kommen kann. Das "Post Combustion"-Phänomen wird auch als stille Oxidation oder Blue Flame-Phänomen bezeichnet. Hierdurch werden Vorgänge beschrieben, bei denen temperaturempfindliche organische Substratmoleküle wie beispielsweise Methacrolein oder andere Aldehyde in gasförmigen, sauerstoffhaltigen Reaktionsmischungen auch bei Temperaturen unterhalb der nominellen Zündtemperatur mit gegenüber Explosionsereignissen verminderter Geschwindigkeit in kürzerkettige Fragmente geringerer Molmasse zerfallen, was von einer spontanen Erhöhung von Druck und Temperatur der Reaktionsmischung begleitet wird. Diese spontanen Druck- und Temperaturereignisse müssen nicht notwendigerweise zum Versagen von Behältern oder Apparaten führen, können jedoch die Deformation von Konstruktionselementen, die Beschädigung von Messeinrichtungen oder das Auslösen von Sicherheitseinrichtungen wie Berstscheiben nach sich ziehen und so eine Unterbrechung des Anlagenbetriebs erzwingen.

Ferner kann ein "Post Combustion"-Phänomen auch als Zündquelle für eine explosionsartige Selbstzündung

der Reaktionsmischung fungieren. Bei einer Selbstentzündung steigt die Temperatur im Reaktionsgas zwischen dem Rohrbodenaustritt des zweiten Oxidationsreaktors und der nachfolgenden Separationsvorrichtung, wie beispielsweise einer Quench-Kolonne, sehr stark an, was zu einer Unterbrechung der Produktion wie z.B. einer Sicherheitsschaltung, führen kann. Je höher die Temperatur des Reaktionsgases ist, desto häufiger treten "Post Combustion"-Phänomene und damit auch Selbstentzündungen auf. Üblicherweise deutet also das Auftreten des "Post Combustion"-Phänomens auf das bevorstehende Lebensende des Katalysators hin.

Die Geschwindigkeit, mit der Katalysator altert, also eine Abnahme seiner Aktivität erleidet, nimmt mit steigender Temperatur zu. Ein Anheben der Salzbadtemperatur führt also dazu, dass ein zeit- und kostenintensiver Austausch des Oxidationskatalysators häufiger durchgeführt werden muss, als wenn die Temperatur konstant gehalten wird. Der Oxidationsreaktor wird folglich bei einer Temperatur betrieben, die es gestattet, einerseits den wirtschaftlichen Betrieb der Oxidationsreaktion zu gewährleisten und andererseits die Alterung des Katalysators auf ein Minimum zu begrenzen. Aus diesem Stand der Technik ergeben sich diverse Probleme, die einer technischen Lösung bedürfen. So kann neben einer temperaturbedingten Abnahme der Aktivität des Kontakt 2 die Aktivität auch durch schädigende Begleitstoffe im Feed der nachgeschalteten Reaktionsstufen hervorgerufen werden.

Ein Beispiel eines solchen Begleitstoffs ist in der ersten Reaktionsstufe nicht vollständig umgesetztes IBEN. Der IBEN-Gehalt im Feed der zweiten Reaktionsstufe kann durch Einhalten eines hohen, nahezu quantitativen Umsatzes in der ersten Reaktionsstufe minimiert werden. Wie bei Nagai et al., Sumitomo Kagaku 2004, 1-12, beschrieben, ist die Erhöhung der IBEN-Toleranz von Kontakten für die Oxidation von MAL ein ständiges Arbeitsfeld der Katalysatorforschung.

Ein zweites Beispiel einer die die Aktivität negativ beeinflussenden Verbindung ist 2-Methyl-2-pentenal, das beispielsweise bei der Herstellung von MAL aus Propanal und Formaldehyd als Nebenprodukt entstehen kann (DE 3508702).

Ein weiteres Beispiel eines die Aktivität von Kontakt 2 beeinträchtigenden Begleitstoffs ist dimeres, oder einfach: di-MAL, das aus MAL durch Diels-Alder-Reaktion gebildet wird:

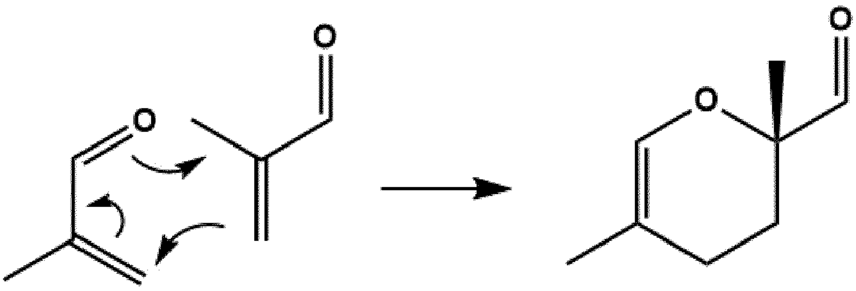

In EP 0194620 wird der negative Einfluss von di-MAL auf die Aktivität eines Kontakt 2 beschrieben. Demnach verringert sich die in der 2. Reaktionsstufe über einem Kontakt des Typs $Mo_{12}V_{0,6}P_{1,2}Cs_2Cu_{0,7}Rh_{0,1}O_x$ erzielte MAS-Ausbeute um 5 %, wenn der Feed 0.4 Massen-% di-MAL enthält.

Es ist bekannt, dass die Lebensdauer eines für die zweite Oxidationsstufe eingesetzten Katalysators dadurch erhöht werden kann, dass die Konzentration der genannten Komponenten im Feed niedrig gehalten wird. So wird in der DE 3508702 ein Verfahren, bei dem die Summe der Konzentration von 2-Methylpentenal und di-MAL im Feed geringer als 0,15 Gew% und die Konzentration von Propanal und Formaldehyd geringer als 1,0 Gew% ist, beschrieben. Die Veröffentlichung gibt jedoch keine Hinweise darauf, wie die Einhaltung dieser Konzentrationsobergrenzen im Betrieb wirtschaftlich realisiert werden kann.

In DE 2143582 wird die Unterdrückung der Bildung oligomerer und polymerer Produkte bei der Warmlagerung von flüssigem Methacrolein, wie z.B. 5 Stunden bei 100 °C beschrieben, wenn das Methacrolein mit einer Mischung aus je 500 ppm Orthophosporsäure und p-tert.-Butylcatechol stabilisiert ist. Nachteilig hieran ist zum einen die große Menge an benötigtem Stabilisator, zum anderen vor allem jedoch die Anwesenheit einer freien Mineralsäure, die unter den Prozessbedingungen Korrosionsschäden nach sich zieht.

Ein weiterer negativer Aspekt der di-MAL-Bildung bei der Lagerung von Methacrolein betrifft die Tatsache, dass das bei der Zwischenlagerung gebildete di-MAL in der zweiten Oxidationsstufe zu hochsiedenden Verbindungen oxidiert wird. Dabei wird in den nachfolgenden Aufarbeitungsschritten dieses abgetrennt und steht somit nicht mehr zur Gewinnung von Methacrylsäure zur Verfügung. In US 2577445 wird ein Verfahren zur Rückspaltung von di-MAL zu MAL beschrieben. In diesem Verfahren wird di-MAL zunächst in sein Semicarbazon umgewandelt und dieses dann pyrolytisch zu MAL gespalten. Nachteilig an dem genannten Vorgehen ist die Einführung eines weiteren Prozessschritts.

Ein Verfahren zur Unterdrückung der di-MAL-Bildung bei der Zwischenlagerung von MAL wird in der JP 2006028067 beschrieben. Hiernach kann die Bildung von di-MAL dadurch vermieden werden, dass MAL bei 0 °C in

einem Lagerbehälter in Gegenwart von 1000 ppm Hydrochinon aufbewahrt wird. Dabei ist der Lagerbehälter nur zu 70% seines Volumens mit MAL befüllt, und die überstehende Gasphase in den verbleibenden 30% des Behältervolumens muss zur Erhaltung der Funktionsfähigkeit des Hydrochinon-Stabilisators mit 6 bis 16 Vol% Sauerstoff angereichert sein. Das beschriebene Verfahren weist durch die Bildung einer Sauerstoff und brennbare Substanzen enthaltenden Mischung im Gasraum des Lagerbehälters den entscheidenden Nachteil eines hohen sicherheitstechnischen Risikos auf. Das Risiko wird insbesondere verschärft, wenn bei Ausfall der Kühlung des Lagerbehälters der MAL-Dampfdruck zunimmt. Nicht beschrieben wird zudem die Auswirkung des hohen Hydrochinon-Gehalts auf die Aktivität der nachfolgenden Reaktionsschritte.

Gemäß den vorstehenden Ausführungen besteht eine Herausforderung also darin, eine geringe Konzentration der die Oxidation von Methacrolein zu Methacrylsäure negativ beeinflussenden Begleitstoffe im Methacrolein-Feed zur nachfolgenden Reaktionsstufe sicherzustellen. Insbesondere muss sichergestellt sein, dass die Bildung von di-MAL während der Zwischenlagerung unterbunden wird.

**Aufgabe**

Aufgabe der vorliegenden Erfindung war es somit, ein neues Verfahren zur Herstellung von Alkylmethacrylaten und optional Methacrylsäure - insbesondere von MMA - zur Verfügung zu stellen, welches von C2- bzw. C4-Bausteinen ausgehend über einen langen Zeitraum unterbrechungsfrei betrieben werden kann.

Eine weitere Aufgabe der vorliegenden Erfindung war es, Alkylmethacrylate und optional Methacrylsäure durch eine Oxidation von aus C2- oder C4-Quellen hervorgehendem Methacrolein zu gewinnen, wobei die für die Oxidation eingesetzten Katalysatoren durch geeignete Verfahrensführung nicht vollständig umgesetzter Rohstoffe und Zwischenprodukte eine verminderte Alterung erfahren.

Eine weitere optionale Aufgabe der vorliegenden Erfindung war es, Alkylmethacrylate, insbesondere Methylmethacrylat, durch die direkte oxidative Veresterung von aus C2- oder C4-Quellen hervorgehendem Methacrolein zu gewinnen, wobei die Alkylmethacrylate einen geringen Gehalt von freiem Methacrolein aufweisen.

Darüber hinaus war es Aufgabe der vorliegenden Erfindung, die von den bei der Oxidation von Methacrolein nicht vollständig umgesetzten Rohstoffen und Zwischenprodukten ausgehenden betrieblichen und sicherheitstechnischen Risiken zu minimieren.

Insbesondere war es Aufgabe der vorliegenden Erfindung, ein Verfahren zur Verfügung zu stellen, bei dem nach Oxidation des Zwischenprodukts Methacrolein zu Methacrylsäure nicht umgesetztes MAL in geeigneter Form abgetrennt und zurückgewonnen wird und hierbei die Bildung von sich negativ auf die nachfolgenden Prozessschritte auswirkenden Nebenprodukten auf ein für diese Prozessschritte unschädliches Konzentrationsniveau reduziert werden kann.

Weitere, nicht explizit genannte Aufgaben, können sich aus den Ansprüchen und der folgenden Beschreibung der Erfindung ergeben, ohne hier explizit aufgeführt zu sein.

**Lösung**

Gelöst werden die genannten Aufgaben durch ein neuartiges Verfahren zur Herstellung von Alkylmethacrylaten und optional Methacrylsäure, insbesondere von Methylmethacrylat (MMA) aus C2- oder C4-Rohstoffen, insbesondere in einem Verfahren mit Methacrolein als Intermediat. Dieses erfindungsgemäße Verfahren zeichnet sich dadurch aus, dass die Lagertemperatur und das Lagervolumen sowie dadurch bedingt die Lagerzeit von Methacrolein gering gehalten werden können.

Als C4-Rohstoff können Substanzen wie Isobuten (IBEN) oder Methyl-tert.-butyleter (MTBE) verwendet werden. MTBE ist ein gebräuchlicher und weit verbreiteter Rohstoff für C4-basierte Prozesse. Er kann leicht transportiert werden und gestattet den Betrieb einer Methacrylsäure oder Alkylmethacrylat-Produktion auch in größerer räumlicher Entfernung von C4-Quellen wie beispielsweise Steamcrackern. IBEN kann aus C4-Strömen durch Veretherung mit Methanol zu MTBE und anschließende Rückspaltung gewonnen werden. C2-basierte Verfahren gehen von Ethylen, welches zunächst zu Propanal und schließlich mit Formaldehyd zu Methacrolein umgesetzt wird, aus.

Das erfindungsgemäße Verfahren basiert dabei auf einem Verfahren, bei dem in einer ersten Reaktionsstufe in einem ersten Reaktor - z.B. Reaktor **A** oder **J** - aus einer C4-Quelle **f2** oder einer C2-Quelle **f6** eine Methacrolein-haltige Fraktion hergestellt wird. Weiterhin wird eine auf der ersten Fraktion basierenden zweite Methacrolein-haltigen Fraktion anschließend in mindestens einem zweiten Reaktor weiter umgesetzt. Dabei ist das Verfahren dadurch gekennzeichnet, dass die Methacrolein-haltige zweite Fraktion in flüssiger Form in einem Zwischenbehälter **D** auf eine Lagertemperatur zwischen -30 °C und 50 °C gekühlt mit einer Verweilzeit kleiner 48 h gelagert wird und von dort in einen Verdampfer **E** oder in flüssiger Form in einen zweiten Reaktor, bei dem es sich um einen Reaktor **G** für eine oxidative Veresterung handelt, geleitet wird.

Die Rückführung des überwiegend Methacrolein enthaltenden Stroms **3b** in den Eingangsstrom vor Reaktor

**B** geschieht dadurch, dass der verflüssigte Strom erfindungsgemäß zunächst einem Zwischenbehälter **D** zugeleitet und darin gelagert wird. Es ist bekannt, dass bei der Lagerung von flüssigen Methacrolein enthaltenden Stoffgemischen Reaktionsprodukte mit höherer Molmasse entstehen können, die sich nachteilig auf die katalytische Aktivität nachfolgender Reaktionsschritte auswirken (siehe z.B. DE 3508702). Ein Vertreter dieser Verbindungen ist beispielsweise das cyclische Dimere des Methacrolein (di-MAL), das aus MAL durch Diels-Alder-Reaktion hervorgeht. Wie im Stand der Technik beschrieben, kann die Bildung von Komponenten wie di-MAL durch Maßnahmen unterdrückt werden, die entweder die Hinzugabe großer Mengen an zum Teil korrosiven Stabilisatoren (siehe z.B. DE 2143582]) oder die Lagerung unter einer sauerstoffhaltigen Atmosphäre beinhalten (siehe z.B. JP 2006028067). Während sich zusätzliche Stabilisatormengen in den Rohrleitungen und Apparaten des Prozesses ablagern können, bringt die Kombination großer Mengen Methacrolein und Sauerstoff enthaltender Gasphasen in Lagerbehältern ein unkalkulierbares Sicherheitsrisiko mit sich.

Überraschenderweise wurde nun gefunden, dass sich die Bildung von die nachfolgenden Reaktionsschritte nachteilig beeinflussenden Nebenprodukten in einfacher, technisch robuster, sicherheitstechnisch risikoarmer und wirtschaftlicher Weise dadurch realisieren lässt, dass bei der Lagerung im Zwischenbehälter **D** Methacrolein nur in geringen Mengen und damit mit geringer Verweilzeit gekühlt gelagert wird. Zusätzlich kann eine gewöhnlich durch die Verwendung großer Puffervolumina für Zwischenprodukte unterstützte stabile Prozessfahrweise nun gerade durch die Minimierung solcher Puffervolumina realisiert werden.

Durch die kurzzeitige Lagerung bei verminderter Temperatur wird die Bildung von Nebenprodukten, insbesondere von di-MAL, unterdrückt. Bevorzugt sind Verfahren, bei denen die Kondensationstemperatur des Methacrolein-haltigen Stroms in der Separationsvorrichtung **C** höher ist als die Lagertemperatur des Methacrolein-haltigen Stroms im Zwischenbehälter **D.** Erfindungsgemäß sind solche Ausführungen des Verfahrens, bei denen die Verweilzeit des Methacrolein-haltigen Stroms im Zwischenbehälters **D** kleiner als 48 h ist und die Lagertemperatur zwischen -30 °C und 50 °C beträgt. Bevorzugt sind solche Ausführungen des Verfahrens, bei denen die Verweilzeit des Methacrolein-haltigen Stroms im Zwischenbehälters **D** kleiner als 12 h, besonders bevorzugt kleiner als 6 h ist und die Lagertemperatur zwischen -20 °C und 30 °C, besonders bevorzugt zwischen -10 °C und 20 °C beträgt.

Die erfindungsgemäße Minimierung des Lagervolumens und der Lagertemperatur von Methacrolein ist neben den beschriebenen Vorteilen mit verschiedenen günstigen sicherheitstechnischen Nebeneffekten verbunden, die eine sichere und unfallfreie Prozessführung unterstützen:

Der erste Nebeneffekt betrifft die hohe inhalative Toxizität von Methacrolein-Dämpfen in Verbindung mit dem hohen Dampfdruck des Methacroleins. Durch die Temperaturabhängigkeit des Dampfdrucks kann im Falle der unbeabsichtigten Freisetzung von Methacrolein dessen Ausbreitungsradius minimiert werden, wenn das Methacrolein gekühlt gelagert wird.

Der zweite Nebeneffekt betrifft die Anfälligkeit hochreaktiver Verbindungen wie Methacrolein, bei der Lagerung sich selbst beschleunigende Reaktionen zu erleiden, die zu einer plötzlichen Druck- und Temperaturerhöhung im Lagerbehälter und infolge zum Behälterversagen führen können. Der dabei entstehende Schaden kann ebenfalls durch die Minimierung des Lagervolumens gering gehalten werden, während die Eintrittswahrscheinlichkeit eine solchen Ereignisses durch die deutliche Unterschreitung der self-accelerating polymerization temperature (SAPT) bei der Lagerung minimiert wird. Die SAPT einer Substanz gibt diejenige untere Temperatur an, ab der eine sich selbst erhaltende Polymerisationsreaktion auftreten kann; sie vereinigt die Einflüsse der Außentemperatur, der Polymerisationkinetik, der Behältergröße und der durch die Wand des Lagertanks auftretenden spezifische Wärmeabfuhr. Die Abhängigkeit der SAPT von der Wärmeabfuhr ist für MAL in ***Tabelle 1*** angegeben. Naturgemäß erhöht sich zusammen mit dem steigenden Oberfläche / Volumen-Verhältnis mit sinkendem Behältervolumen die spezifische Wärmeabfuhr, womit die im Hinblick auf Polymerisationsereignisse als sicher anzusehende Lagertemperatur mit sinkendem Volumen des Lagertanks steigt.

*Tabelle 1: Self accelerating polymerization temperature von Methacrolein in Abhängigkeit von der Wärmeabfuhr des Lagertanks*

| Spezifische Wärmeabfuhr [mW/(kg*K)] | SAPT [°C] |
|---|---|
| 60 | 25 |
| 80 | 30 |
| 100 | 35 |
| 150 | 35 |
| 200 | 40 |

Der dritte Nebeneffekt betrifft die Tatsache, dass solche selbstbeschleunigenden Reaktionen typischerweise durch die Zugabe von Stabilisatoren unterbunden werden. Durch die Kreislaufführung von nicht umgesetztem Methacrolein kommt es jedoch zu einer Ablagerung solcher Stabilisatoren in Form von Feststoffen, die mit hohem Aufwand bei Stillständen aus der Produktionsanlage entfernt werden müssen. Eine geringe Lagertemperatur gestattet die Absenkung der Stabilisatorkonzentration und minimiert dadurch die Ablagerungen.

Ein weiterer positiver Effekt der erfindungsgemäßen kurzzeitigen gekühlten Zwischenlagerung von Methacrolein ergibt sich durch die hohe Reaktivität des di-MAL in nachfolgenden Reaktionsschritten.

Weiterhin ist es bevorzugt, dass der nach Aufarbeitung und Abtrennung des überwiegend Methacrylsäure enthaltenden Stroms 3a erhaltene überwiegend Methacrolein-haltige Stoffstrom 3b derart kondensiert und vorgelagert wird, dass die Konzentration von Methacrolein-Dimeren (di-MAL) im flüssigen Feedstrom 4 zum Verdampfer E bzw. - wie weiter unten beschrieben - zu Reaktor G einen Gehalt von 1 Gew% nicht überschreitet.

In einer ersten erfindungsgemäßen Ausführungsform wird ausgehend von einer C4-Quelle f2 in dem ersten Reaktor A ein Methacrolein-haltiges Prozessgas 1 als erste Methacrolein-haltige Fraktion gebildet. Dieses wird darauf in Form eines Prozessgases **1** nach optionaler Zumischung eines Sauerstoff und Wasserdampf enthaltenden Gasgemischs **6** in ein Prozessgas **7** überführt, welches in einer zweiten Reaktionsstufe in einem Reaktor **B** durch Partialoxidation in der Gasphase zu einem Methacrylsäure enthaltenden Prozessgas **2** umgesetzt wird.

Dabei wird in Reaktor **A** die C4-Quelle bei Temperaturen zwischen 320 bis über 400 °C bei leichtem Überdruck in Anwesenheit von Luftsauerstoff und Wasserdampf, sowie optional einem im späteren Aufarbeitungsteil anfallenden Recycle-Gas zu Methacrolein oxidiert. Der Umsatz liegt beim Tandemverfahren bei größer 98%. Üblicherweise liegt die Verweilzeit im Rohrbündelreaktor mit modernen dotierten Bismut-Molybdat-Kontakten, wie beispielsweise in der US 59,292,75 beschrieben, bei 1 bis 4 Sekunden,. Es werden GHSV-Werte zwischen 1000 und 2000 $s^{-1}$ erreicht. Das austretende Methacrolein-haltige Prozessgas **1** wird mit einem kühleren Mischstrom **5** enthaltend eine Recycle-MAL Gasphase sowie dem aus einem Luftsauerstoff enthaltenden Gasgemisch **f3** und einem Wasserdampf enthaltenden Gasgemisch **f4** hervorgehenden Gasgemisch **6** vermischt. Somit ergibt sich das Feedgas **7** der zweiten Oxidationsstufe. Optional können das Luftsauerstoff enthaltende Gasgemisch f3 und/oder das Wasserdampf enthaltende Gasgemisch f4 zur Verdampfung des methacroleinhaltigen Stroms 4 ebenfalls in den Verdampfer E eingebracht werden. Die zweite Oxidationsstufe in Reaktor **B** wird wie die erste bei einem moderaten Überdruck zwischen 0,1 und 2 bar und bei Temperaturen zwischen 260 und 360 °C betrieben. Hierzu kommen Heteropolysäurekontakte auf Basis von Molybdän und Phosphor sowie einigen weiteren Dopanten zum Einsatz (wie z.B. in US 2007/0010394 beschrieben). Die modifizierten Heteropolysäuren weisen bei höheren Umsätzen zunehmend schlechtere Selektivitäten zu Methacrylsäure auf. Aus diesem Grund wird der Umsatz und die damit verbundene Katalysatorbelastung bevorzugt zwischen 65% und 85% eingestellt. Aus dieser Tatsache ergibt sich für alle C4-basierten Verfahren wie auch für diese Ausführungsform des erfindungsgemäßen Verfahrens die Notwendigkeit, nicht umgesetztes MAL aus dem nach Reaktor **B** resultierenden Prozessgas **2** vom gewünschten Produkt Methacrylsäure abzutrennen und letztlich als sogenanntes "Recycle-MAL" vor den zweiten Oxidationsreaktor **B** zurückzufahren.

In bevorzugten Ausführungen dieser Ausführungsform folgen darauf diese Schritte:

a. Das Prozessgas **2** wird in einer Separationsvorrichtung **C** in einen überwiegend Methacrylsäure enthaltenden Strom **3a** und einen überwiegend Methacrolein enthaltenden Strom **3b** aufgetrennt.
b. Die in Schritt a beschriebene Separationsvorrichtung umfasst jeweils mindestens einen der Schritte Quenchen, Kristallisation und Destillation.
c. Der überwiegend Methacrolein-haltige Strom **3b** am Kopf einer fraktionierenden Destillationskolonne wird kondensiert und der kondensierte Methacrolein-haltige Strom **3b** danach in den Zwischenbehälter **D** geleitet.
d. Aus Zwischenbehälter **D** wird anschließend ein thermostatisierter, insbesondere gekühlter Methacrolein-haltiger Strom **4** in den Verdampfer **E** geleitet, um dort Strom **4**, optional unter Beteiligung eines Luftsauerstoff enthaltenden Gasgemischs **f3** und/oder eines Wasserdampf enthaltenden Gasgemischs **f4** in einen Methacrolein-haltigen Gasstrom **5** zu überführen, der zusammen mit Gasgemisch **6**, optional gemischt zu einem Gasstrom **7**, in Reaktor **B** geleitet wird.

Das in der zweiten Reaktionsstufe im Reaktor **B** gebildete Prozessgas kann darauf in einer nachfolgenden Separationsvorrichtung **C** aufgetrennt werden. Dabei erfolgt eine Auftrennung in einen überwiegend Methacrylsäure enthaltenden Strom **3a** und einen überwiegend Methacrolein enthaltenden Strom **3b**. Die Auftrennung ist nötig, um den in Reaktor **B** nicht umgesetzten Teil des Methacroleins durch Rückführung vor den Reaktor möglichst vollständig in das gewünschte Zielprodukt Methacrylsäure zu überführen. Die der Separationsvorrichtung **C** zugrundeliegenden Trennverfahren umfassen jeweils mindestens einen der Schritte Quenchen, Kristallisation und Destillation, und der überwiegend Methacrolein enthaltende Strom **3b** wird durch Kondensation am Kopf einer fraktionierenden Destillationskolonne erhalten.

Das heiße Prozessgas **2** aus Reaktor **B** tritt üblicherweise bei 250 bis 360°C aus dem Reaktor aus und muss zunächst abgekühlt werden. Üblicherweise wird zunächst über einen rekuperativen Gaskühler auf eine Temperatur zwischen 150 und 250 °C abgekühlt. Rekuperative Gaskühler sind bevorzugt, weil mit diesen die Wärme zur Dampfgenerierung genutzt wird. Danach wird die jetzt in der Temperatur reduzierte Gasphase üblicherweise bei Temperaturen zwischen 50 bis 100 °C in eine umlaufende kondensierte Quenchphase geleitet. Diese Quenchphase kann der Sumpfteil einer Quenchkolonne sein, die über eine Pumpe umgewälzt und thermostatisiert wird. Am Kopf dieser Quenchkolonne geht der größte Teil des Methacroleins gasförmig zusammen mit dem Prozessgas über, während der Großteil der gebildeten Methacrylsäure im Sumpf kondensiert und gequencht wird. In einem nächsten Verfahrensschritt wird bevorzugt Methacrolein kondensiert bzw. zusammen mit Wasser absorbiert. In diesem Schritt fällt Methacrolein, zusammen mit allen kondensierbaren Nebenkomponenten, wie z.B. Leichtsiedern, flüssig an. Trotzdem erreicht man eine effektive Abtrennung vom Prozessgas, das am Kopf dieser Kolonne entweicht. In einem letzten Schritt kann Methacrolein jetzt aus der Absorberphase desorbiert werden und man gelangt so zum überwiegend Methacrolein enthaltenden Strom **3b**, welcher einen MAL-Gehalt von größer 70 Gew.% aufweist.

Aus dem überwiegende Methacrylsäure enthaltenden Strom **3a** kann nach geeigneter Aufarbeitung ein Methacrylsäure in reiner Form enthaltender Strom **p1** gewonnen werden. Besonders bevorzugt erfolgt diese Aufarbeitung destillativ und/oder extraktiv.

In einer alternativen weiteren Ausführungsform dieser ersten Ausführungsform des erfindungsgemäßen Verfahrens wird der überwiegend Methacrylsäure enthaltende Strom **3a** einem Reaktor **F**, bei dem es sich insbesondere um einen Veresterungsreaktor handelt, zugeführt, wo die Methacrylsäure, bevorzugt sauer katalysiert, zusammen mit einem einen geeigneten Alkohol enthaltenden Strom **f8** zu einem ein Alkylmethacrylat enthaltenden Produktstrom **p2** umgesetzt wird. Entsprechende Prozessparameter zur Gewinnung reiner Methacrylsäure bzw. reinen Alkylmethacrylaten können z.B. der EP 19 952 32 entnommen werden.

Ein solchermaßen erhaltener gekühlter überwiegend Methacrolein enthaltender Stoffstrom **4** wird in dieser ersten Ausführungsform bevorzugt dadurch dem Oxidationsprozess wieder zugeleitet, dass er zunächst in einem Verdampfer **E** zu einem Gasstrom **5** verdampft wird. Optional kann dieser Gasstrom **5** mit einem aus einem Sauerstoff enthaltenden Gasstrom **f3** und einem Wasserdampf enthaltenden Gasstrom **f4** hervorgehenden Gasgemisch **6** zu einem Prozessgases **7** vereinigt werden, um als solches anschließend dem Prozessgas 1 vor Reaktor **B** zugemischt zu werden. Bevorzugt sind dabei solche Ausführungen des Prozesses dieser Ausführungsform, bei denen im Prozessgas **1** der Isobuten-Gehalt einen Wert von 2000 Vol-ppm nicht übersteigt und Aufarbeitung, Thermostatisieren bzw. Zwischenkühlung und erneute Verdampfung des Methacrolein-haltigen Prozessgases einen Methacrolein-haltigen Strom **5** ergibt, der, bezogen auf Methacrolein, 0,2 bis 25 Gew%, bevorzugt bis 20 Gew% weitere carbonylische $C_1$-$C_4$-Kohlenwasserstoffe enthält. Weiterhin ist es bevorzugt, dass dieses Prozessgas einen Gehalt an Methacrolein-Dimeren von kleiner 1 Gew% aufweist. Durch den bedingt durch geringe Lagermengen und/oder geringe Lagertemperaturen ermöglichten geringen Gehalt an di-MAL im Methacrolein-haltigen Strom **5** und damit auch im Eingangsstrom zu Reaktor **B** kann nachweislich die Reaktionstemperatur in Reaktor **B** länger auf einem niedrigen Niveau gehalten werden. Eingedenk des negativen Einflusses der Reaktortemperatur auf die Lebenszeit eines Methacrolein-Oxidationskatalysators kann somit überraschend eine Lebenszeitverlängerung des Katalysators dadurch bewirkt werden, dass zwischengespeichertes MAL bei möglichst geringen Temperaturen für möglichst geringe Zeitspannen gelagert wird.

Soll bei der Herstellung von Alkylmethacrylaten nicht von C4-Quellen ausgegangen werden, kann in einer zweiten, sehr bevorzugten Ausführungsform der vorliegenden Erfindung das erfindungsgemäße Verfahren ausgehend von einer C2-Quelle **f6**, bevorzugt Ethylen, durchgeführt werden. Hier wird die aus z.B. Ethylen hergestellte Zwischenstufe Propanal in einen aufgereinigten flüssigen Methacrolein enthaltenden Strom **13** umgewandelt. Insbesondere wird in dieser Ausführungsform ausgehend von der C2-Quelle **f6** in einem Reaktor H unter Entstehung eines Propionaldehyd-haltigen Stroms **8**, der mittels mindestens eine Destillation umfassenden Reinigungsschritts **I** aufgearbeitet wird, ein Propionaldehyd enthaltender Strom **10** gebildet. Dieser Strom wird daraufhin in einem nachfolgenden Reaktor **J** und einem weiteren nachfolgenden Reinigungsschritt **K** zu einem Methacrolein-haltigen Strom **13** umgewandelt wird und dieser Methacrolein-haltigen Strom **13** schließlich in den Zwischenbehälter **D** geleitet.

Alternativ kann der Strom **13** auch direkt im Verdampfer **E** in die Gasphase überführt werden. In dieser Variante wird nur das MAL des Stroms **3b** in den Zwischenbehälter **D** geleitet und dort gelagert.

Dafür wird zunächst Ethylen zusammen mit einem Synthesesgasstrom **f5** in einem Reaktor **H** zu Propanal hydroformyliert. Die für die Umsetzung von Ethylen in Propanal durchgeführte Hydroformylierungsreaktion ist in der Standardliteratur ausführlich beschrieben, so z.B. in E. Billig, D. Bryant, Kirk-Othmer Encyclopedia of Chemical Technology, John Wiley & Sons, Inc., OXO Process und R. Franke et al., Applied Hydroformylation, dx.doi.org/10.1021/cr3001803, Chem. Rev. 2012, 112, 5675-5732.

Im Allgemeinen werden für diese Reaktion Katalysatoren eingesetzt. Zu den bevorzugten Katalysatoren zählen insbesondere Verbindungen, die Rhodium, Iridium, Palladium und/oder Cobalt umfassen, wobei Rhodium besonders bevorzugt ist. Gemäß einer besonderen Ausführungsform können insbesondere Komplexe, die mindestens eine phosphorhaltige Verbindung als Ligand umfassen, zur Katalyse eingesetzt werden. Bevorzugte phosphorhaltige Verbindungen umfassen aromatische Gruppen und mindestens ein, besonders bevorzugt zwei Phosphoratome. Zu den phosphorhaltigen Verbindungen zählen insbesondere Phosphine, Phosphite, Phosphinite, Phosphonite. Beispiele für Phosphine sind Triphenylphosphin, Tris(p-tolyl)phosphin, Tris(m-tolyl)phosphin, Tris(o-tolyl)phosphin, Tris(p-methoxyphenyl)phosphin, Tris(p-dimethylaminophenyl)phosphin, Tricyclohexylphosphin, Tricyclopentylphosphin, Triethylphosphin, Tri-(1-naphthyl)phosphin, Tribenzylphosphin, Tri-n-butylphosphin, Tri-t-butylphosphin. Beispiele für Phosphite sind Trimethylphosphit, Triethylphosphit, Tri-n-propylphosphit, Tri-i-propylphosphit, Tri-n-butylphosphit, Tri-i-butylphosphit, Tri-t-butylphosphit, Tris(2-ethylhexyl)phosphit, Triphenylphosphit, Tris(2,4-di-t-butylphenyl)phosphit, Tris(2-t-butyl-4-methoxyphenyl)phosphit, Tris(2-t-butyl-4-methylphenyl)phosphit, Tris(p-kresyl)phosphit. Beispiele für Phosphanite sind Methyldiethoxyphosphin, Phenyldimethoxyphosphin, Phenyldiphenoxyphosphin, 2-Phenoxy-2H-dibenz[c,e][1,2]oxaphosphorin und dessen Derivate, in denen die Wasserstoffatome ganz oder teilweise durch Alkyl- und/oder Arylreste oder Halogenatome ersetzt sind. Gängige Phosphinitliganden sind Diphenyl(phenoxy)phosphin und dessen Derivate Diphenyl(methoxy)phosphin und Diphenyl(ethoxy)phosphin.

Katalysatoren und Liganden zur Hydroformylierung werden beispielsweise in WO 2010/030339 A1, WO 2008/071508 A1, EP 982314 B1, WO 2008/012128 A1, WO 2008/006633 A1, WO 2007/036424 A1, WO 2007/028660 A1, WO 2005/090276 A1 dargelegt, wobei auf diese Druckschriften zu Offenbarungszwecken verwiesen wird und die darin offenbarten Katalysatoren und Liganden von dieser Anmeldung umfasst werden. In diesen Druckschriften werden auch Reaktionsbedingungen dargelegt. Zur Hydroformylierung von Ethen werden Kohlenmonoxid und Wasserstoff üblicherweise in Form eines Gemisches, dem sogenannten Synthesegas, eingesetzt. Die Zusammensetzung des zur Hydroformylierung eingesetzten Synthesegases kann in weiten Bereichen variieren. Das molare Verhältnis von Kohlenmonoxid und Wasserstoff beträgt in der Regel 2:1 bis 1:2, insbesondere etwa 45:55 bis 50:50. Die Temperatur bei der Hydroformylierungsreaktion liegt im Allgemeinen in einem Bereich von etwa 50 bis 200 °C, vorzugsweise etwa 60 bis 190 °C, insbesondere etwa 90 bis 190 °C. Die Umsetzung wird vorzugsweise bei einem Druck im Bereich von etwa 5 bis 700 bar, vorzugsweise 10 bis 200 bar, insbesondere 15 bis 60 bar durchgeführt. Der Reaktionsdruck kann in Abhängigkeit von der Aktivität des eingesetzten Hydroformylierungskatalysators variiert werden. Geeignete druckfeste Reaktionsapparaturen für die Hydroformylierung sind dem Fachmann bekannt. Dazu zählen die allgemein üblichen Reaktoren für Gas-Flüssig-Reaktionen, wie z. B. Gasumlaufreaktoren, Blasensäulen etc., die gegebenenfalls durch Einbauten unterteilt sein können. Weitere bevorzugte Ausgestaltungen einer Hydroformylierungsreaktion sind unter anderem in EP 12 946 68 dargelegt, wobei der Inhalt dieser Druckschrift durch Referenz hierauf in die vorliegende Anmeldung eingefügt wird.

Nach dem Hydroformylierungsschritt wird der flüssiger überwiegend Propionaldehyd enthaltende Prozessstrom **8** in einer Separationsvorrichtung **I** in einen flüssigen überwiegend den Hydroformylierungskatalysator enthaltenden Prozessstrom **9** und einen aufgereinigten flüssigen Propionaldehyd enthaltenden Strom **10** aufgetrennt, wobei der flüssige überwiegend den Hydroformylierungskatalysator enthaltende Prozessstrom **9** wieder in den Hydroformylierungsreaktor **H** zurückgeführt wird. Geeignete Ausführungsformen für eine solche Separationsvorrichtung sind beispielsweise in der WO 2007/036424 A1 oder der EP 12 946 68 beschrieben.

Der aufgereinigte flüssige Propionaldehyd enthaltende Strom **10** wird anschließend einem Reaktor **J** zugeführt, wo er zusammen mit einem Formalin enthaltenden Strom **f7** in einer Mannich-artigen Reaktion in einen flüssigen überwiegend Methacrolein enthaltenden Prozessstrom **11** überführt wird. Bevorzugte Verfahren zur Herstellung von Methacrolein, ausgehend von Propanal und Formalin sind unter anderem in den Druckschriften US 71,41702, DE 3213681, US 44,080,79, US 28,484,99; JPH 04173757 A, JP 3069420 B2 und EP 03 179 09 beschrieben.

Die Umsetzung von Propanal mit Formaldehyd wird in Gegenwart von organischen Basen, vorzugsweise Aminen und gleichzeitig Säuren durchgeführt, wobei es sich bei den Säuren, in der Regel um anorganische Säuren handelt. Beispiele dafür sind organischer Mono-, Di- bzw. Polycarbonsäure, bevorzugt Monocarbonsäure, insbesondere aliphatischer Monocarbonsäure. Als Carbonsäuren verwendet man zweckmäßig aliphatische Monocarbonsäuren mit 1 bis 10, vorzugsweise 2 bis 4 Kohlenstoffatomen, oder Di- und Polycarbonsäuren mit 2 bis 10, vorzugsweise 2 und 4 bis 6 Kohlenstoffatomen. Die Dicarbonsäuren und Polycarbonsäuren können aromatische, araliphatische und bevorzugt aliphatische Carbonsäuren sein. Geeignet sind z.B. Essigsäure, Propionsäure, Methoxyessigsäure, n-Buttersäure, iso-Buttersäure, Oxalsäure, Bernsteinsäure, Weinsäure, Glutarsäure, Adipinsäure, Maleinsäure, Fumarsäure. Andere organische Säuren sind prinzipiell ebenfalls verwendbar, in der Regel aber aus Preisgründen weniger zweckmäßig. Als anorganische Säuren werden in der Regel Schwefel- und Phosphorsäure eingesetzt. Es können auch Säuregemische verwendet werden. Besonders bevorzugt wird zur Umsetzung von Propanal und Formaldehyd mindestens eine organische Säure, besonders bevorzugt Essigsäure eingesetzt. Der Anteil an Säure beträgt zwischen 0,1 und 20, vorteilhaft von 0,5 bis 10, bevorzugt 1 bis 5 Mol-%, bezogen auf Propanal.

Bevorzugte organische Basen sind insbesondere sekundäre Amine. Als solche Amine kommen vorzugsweise Amine der Formel $R_1R_2NH$, in Betracht, bei denen $R_1$ und $R_2$ gleich oder verschieden sind und jeweils einen Alkylrest mit 1 bis 10, vorteilhaft 1 bis 8, insbesondere 1 bis 4 Kohlenstoffatomen, die noch durch Ether-, Hydroxy-, sekundäre, tertiäre Aminogruppen, insbesondere durch 1 bis 2 dieser Gruppen, substituiert sein können, einen Aralkylrest mit 7 bis 12 Kohlenstoffatomen, einen Cycloalkylrest mit 5 bis 7 Kohlenstoffatomen bedeuten, R1 und R2 auch mit dem benachbarten Stickstoff Glieder eines heterocyclischen, vorteilhaft 5 bis 7-gliedrigen Ringes, der noch ein weiteres Stickstoffatom und/oder ein Sauerstoffatom enthalten und durch Hydroxyalkyl- oder Alkylgruppen mit 1 bis 4 Kohlenstoffatomen substituiert sein kann, bezeichnen können. Als Amine kommen beispielsweise in Betracht: Dimethylamin, Diethylamin,

Methylethylamin, Methylpropylamin, Dipropylamin, Dibutylamin, Di-iso-propylamin, Di-iso-butylamin, Methyl-iso-propylamin, Methyl-iso-butylamin, Methyl- sek.butylamin, Methyl-(2-methylpentyl)-amin, Methyl-(2-ethylhexyl)-amin, Pyrrolidin, Piperidin, Morpholin, N-Methylpiperazin, N-Hydroxyethyl-piperazin, Piperazin, Hexamethylenimin, Diethanolamin, Methylethanolamin, Methylcyclohexylamin, Methylcyclopentalam in, Dicyclohexylam in oder entsprechende Gemische. Ferner kann vorgesehen sein, dass mindestens eines der eingesetzten Amine keine Hydroxygruppe aufweist. Besonders bevorzugt beträgt der Anteil an Aminen mit mindestens einer Hydroxygruppe höchstens 50 Gew%, vorzugsweise höchstens 30 Gew%, und besonders bevorzugt höchstens 10 Gew%, bezogen auf das Gewicht der eingesetzten Amine. Der Anteil an organischer Base, vorzugsweise sekundären Aminen beträgt zwischen 0, 1 und 20, vorteilhaft von 0,5 bis 10, bevorzugt 1 bis 5 Mol%, bezogen auf Propanal. Das Verhältnis der Äquivalente Amin zu Säure wird vorzugsweise so gewählt, dass im Reaktionsgemisch vor der Reaktion ein pH-Wert von 2,5 bis 9 resultiert. Ferner kann vorgesehen sein, dass das molare Verhältnis von Säure zu organischer Base, vorzugsweise Amin im Bereich von 20:1 bis 1:20, bevorzugt im Bereich von 10:1 bis 1:10, besonders bevorzugt im Bereich von 5:1 bis 1:5 und speziell bevorzugt im Bereich von 2:1 bis 1:2 liegt. Die Reaktionstemperatur der Umsetzung von Propanal mit Formaldehyd am Austritt der Reaktionszone liegt zwischen 100 und 300 °C, vorzugsweise 130 und 250 °C, bevorzugt von 140 bis 220 °C, insbesondere 150 bis 210 °C. Der Reaktionsdruck liegt im Bereich von 2 bis 300, bevorzugt bei 5 bis 250, besonders bevorzugt von 10 bis 200 bar, vorteilhaft von 15 bis 150 bar, bevorzugt 20 bis 100 bar und insbesondere 40 bis 80 bar. Druck und Temperatur werden so eingestellt, dass die Umsetzung stets unterhalb des Siedepunktes des Reaktionsgemisches erfolgt, die Reaktion also in flüssiger Phase verläuft. Alle Druckangaben im Rahmen der vorliegenden Anmeldung erfolgen als absoluter Druck in der Maßeinheit bar. Die Verweilzeit bzw. Reaktionszeit beträgt vorzugsweise höchstens 25, zweckmäßig 0,01 bis 25, vorteilhaft 0,015 bis 10, bevorzugt 0,03 bis 2 Minuten. Besonders bevorzugt liegt die Verweilzeit bzw. Reaktionszeit im Bereich von 0,1 bis 300 Sekunden, speziell bevorzugt im Bereich von 1 bis 30 Sekunden. Als Reaktor wird bei Verweilzeiten unter 10 Minuten vorteilhaft ein Rohrreaktor eingesetzt. Die Verweilzeit bezieht sich hierbei auf die Zeit, über die die Reaktionsmischung umgesetzt wird. Hierbei liegen sämtliche Komponenten bei Reaktionsdruck und Temperatur vor, so dass diese Zeit aus der Strecke zwischen Mischpunkt und dem Entspannungspunkt berechnet werden kann. Der Entspannungspunkt ist der Punkt, bei dem die Mischung vom Reaktionsdruck auf einen Druck unterhalb von 5 bar gebracht wird. In den Reaktionsgemischen können neben Wasser zusätzlich organische Lösungsmittel wie z.B. Propanol, Dioxan, Tetrahydrofuran, Methoxyethanol enthalten sein.

Ferner kann vorgesehen sein, dass die Umsetzung von Propanal mit Formaldehyd zu Methacrolein im Reaktor **J** in Gegenwart von bevorzugt mindestens 0,1 Gew%, vorzugsweise mindestens 0,2 Gew% und besonders bevorzugt mindestens 0,5 Gew% Methanol bezogen auf Formalin erfolgt. Trotz dieser höheren Methanol-Konzentrationen kann aufgrund der erfindungsgemäßen Reaktionsführung für den optionalen Folgeschritt **G** der Direkten Oxidativen Veresterung auf eine aufwendige Abtrennung von Methanol auf der Stufe der Formalinherstellung und/oder der Methacroleinreinigung verzichtet werden. Gemäß einer besonderen Ausgestaltung kann Formaldehyd und Propanal gemischt werden, bevor diese Edukte auf Reaktionsdruck und/oder Temperatur gebracht werden.

Die Reaktion kann in Reaktor **J** wie folgt durchgeführt werden: Ein Gemisch von Propanal, Amin, Formaldehyd und zweckmäßig Wasser und/oder Säure und/oder Base wird während der Reaktionszeit bei der Reaktionstemperatur und dem Reaktionsdruck gehalten. In einer bevorzugten Ausführungsform kann ein Gemisch (zweckmäßig äquimolares Gemisch) aus Formaldehyd und Propanal über einen Wärmetauscher auf die gewünschte Reaktionstemperatur erhitzt und einem Rohrreaktor zugeleitet werden. In dieses Gemisch kann eine Katalysatorlösung (Lösung des sekundären Amins und einer Säure, zweckmäßig in $H_2O$) am Reaktoreingang eingedüst werden, die gegebenenfalls über einen Wärmetauscher ebenfalls auf die Reaktionstemperatur erwärmt wird. Die stark exotherme Reaktion setzt ein und das Reaktionsgemisch erwärmt sich weiter. Der Druck, unter dem die Reaktion abläuft, wird vorzugsweise durch ein Druckhalteventil am Ausgang des Reaktors **J** auf solchen Werten gehalten, dass das Reaktionsgemisch auch bei hohen Temperaturen im Reaktor während der Reaktionszeit noch flüssig bleibt. Nach der Umsetzung kann das Reaktionsgemisch auf Normaldruck entspannt und in der Separationsvorrichtung **K** aufgearbeitet werden. Bei der Herstellung von Methacrolein aus Propanal und Formaldehyd wird bevorzugt das Reaktionsgemisch einer Kolonne zugeleitet und dort mit Wasserdampf gestrippt. Das Methacrolein verlässt zusammen mit Wasser die Kolonne am Kopf. Das Gemisch wird kondensiert und über ein Phasentrenngefäß in eine obere und eine untere Phase getrennt. Die obere Phase wird als aufgereinigter flüssiger Methacrolein enthaltender Strom in den zuvor beschriebenen Zwischenbehälter **D** bzw. alternativ in den zuvor beschriebenen Verdampfer **E** geführt. Die untere Phase besteht hauptsächlich aus Wasser. Vorzugsweise kann sie zur Entfernung des noch darin gelösten Methacroleins zumindest teilweise wieder in die Kolonne zurückgeführt werden. Die wässrige Katalysatorlösung kann am Sumpf der Kolonne zusammen mit dem bei der Reaktion gebildeten Wasser und dem Wasser der Formaldehydlösung abgezogen werden. Für die Weiterverarbeitung kann, wenn sehr wenig Amin und/oder Säure eingesetzt wird und deshalb die Rückführung des Katalysators nicht mehr lohnt, die Sumpfflüssigkeit verworfen werden. Bei größeren Amin- und/oder Säurekonzentrationen im Sumpfablauf kann aber auch Wasser teilweise destillativ abgetrennt und als flüssiger überwiegend das Mannich-Katalysatorsystem enthaltender Prozessstrom **12** wieder in den Reaktor zurückgeführt werden. Außerdem ist es möglich, den Sumpfablauf so in zwei Teilströme aufzuteilen, dass ein Teilstrom gerade die Menge an Wasser mit sich führt, die bei der Reaktion gebildet und

mit den Ausgangsstoffen eingegeben wurde. Dieser Teilstrom wird dann ausgeschleust und der restliche Anteil in den Reaktor zurückgeführt. Wässriger Formaldehyd und Propanal können auch getrennt vorgeheizt und dem Reaktor zugefahren werden.

Gemäß einer besonders bevorzugten Ausführungsform kann die Herstellung von Methacrolein aus Propanal und Formaldehyd in einer Tandemreaktion erfolgen, wobei Propanal durch die Umsetzung von Ethylen, Kohlenmonoxid und Wasserstoff gemäß den Schritten **H** und **I** erhalten und unmittelbar mit Formaldehyd gemäß den Schritten **J** und **K** umgesetzt wird. Dieses Verfahren wird ausführlich von Deshpande et al., Biphasic catalysis for a selective oxo-Mannich tandem synthesis of methacrolein, Journal of Molecular Catalysis A: Chemical 211 (2004) 49-53, doi:10.1016/j.molcata.2003.10.010 und in US 71,417,02 beschrieben.

Weiterhin gibt es zwei weitere Ausführungsformen der vorliegenden Erfindung, wobei es sich bei der dritten Ausführungsform um eine Variation der ersten Ausführungsform handelt, in der das C4-basierte MAL nicht dem Verdampfer E zur späteren Gasphasenoxidation zu Methacrylsäure zugeführt wird, sondern in einen Reaktor G geleitet wird, wo eine oxidativer Veresterung erfolgt.

Analog bezieht sich die vierte Ausführungsform auf MAL, welches auf der Herstellung gemäß dem ersten Teil der zweiten Ausführungsform aus C2-Rohstoffen basiert.

In diesen beiden weiteren Ausführungsformen des erfindungsgemäßen Verfahrens kann der nach dem Zwischenbehälter **D** erhaltene gekühlte überwiegend MAL enthaltende Strom **14** einem Reaktor **G** zugeführt werden, wo er mit einem einen Alkohol enthaltenden Strom **f9** und einer sauerstoffhaltigen Gasmischung **f10** einer direkten oxidativen Veresterungsreaktion (Direct Oxidative Esterification, DOE) unterzogen wird. Die DOE-Reaktion verläuft in flüssiger Phase und ergibt aus MAL und dem Alkohol in einem Reaktionsschritt den das entsprechende Alkylmethacrylat enthaltenden Produktstrom **p3**. Das im MAL-Feed enthaltene di-MAL enthält ebenfalls eine Carbonylgruppe und kann daher ebenfalls zum entsprechenden Alkylester umgewandelt werden:

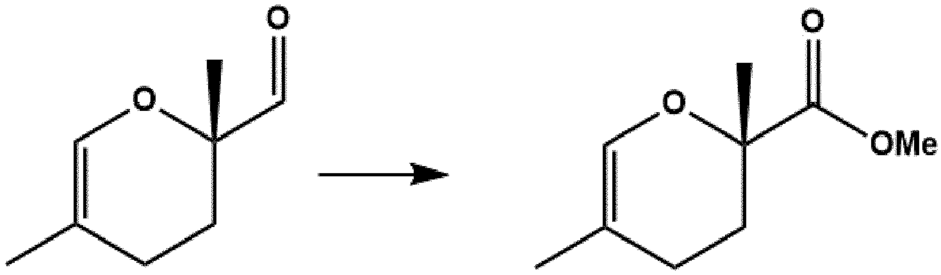

Es hat sich gezeigt, dass di-MAL in der DOE-Reaktion eine höhere Reaktivität aufweist als MAL und so bevorzugt zum entsprechenden Alkylester umgewandelt wird. Eine geeignete Aufarbeitungssequenz für via DOE gewonnenes Alkylmethacrylat sieht zunächst die destillative Abtrennung des gegenüber dem Alkylmethacrylat niedriger siedenden überschüssigen Alkohols bei geringerer Temperatur und anschließend die destillative Abtrennung des Alkylmethacrylats von höher siedenden Nebenprodukten bei höherer Temperatur vor (siehe dazu z.B. WO 2014 170223). Im ersten Destillationsschritt verbleiben also di-MAL mit einem Siedepunkt von 171 °C sowie di-MAL-Methylester mit einem Siedepunkt von 204 °C gemeinsam mit MMA mit einem Siedepunkt von 100 °C im Sumpfprodukt und gehen gemeinsam in den zweiten Trennschritt über, während überschüssiges Methanol (Siedepunkt von 65 °C) über Kopf entfernt wird. Im zweiten, bei höherer Temperatur durchgeführten Trennschritt kann der im Alkylmethacrylat enthaltene di-MAL-Alkylester durch die thermische Belastung zu einem Äquivalent MAL und einem Äquivalent Alkylmethacrylat gespalten werden. In der DOE-Reaktion nicht umgesetztes di-MAL wird in gleicher Weise zu zwei Äquivalenten MAL gespalten. Es besteht somit das Risiko, auf diese Weise ein mit MAL verunreinigtes Alkylmethacrylat zu erhalten. Aus der EP 34 504 22 ist bekannt, dass beispielsweise die Anwesenheit von freiem Methacrolein in MMA die Polymerisations-Eigenschaften von MMA negativ beeinflusst. Ein weiterer Vorteil der erfindungsgemäßen kurzzeitigen und kalten Zwischenlagerung von MAL besteht somit darin, dass der Polymerisationsprozess von aus solchermaßen gelagertem MAL via DOE gewonnenen MMA erleichtert wird. Weiterhin sei angemerkt, dass di-MAL selbst, sowie der daraus gebildete Methylester im Endprodukt, insbesondere in PMMA zu unerwünschten Gelbfärbungen führen können.

Die folgenden Beispiele dokumentieren die Unterbindung der Bildung von Nebenproduktion durch geeignete Lagerung von Methacrolein und den günstigen Effekt von nebenproduktarmem Methacrolein auf die Leistungsfähigkeit des Methacrolein-Oxidationskatalysators. Weitere Bespiele demonstrieren die Umsetzung des Nebenprodukts Di-MAL zu Di-MAL-Methylester bei der Direkten Oxidativen Veresterung (DOE) von MAL zu MMA und somit den Transport von hochsiedenden MAL-Äquivalenten in die Aufarbeitungssequenz eines MMA-DOE-Verfahrens.

**Bezugszeichenliste**

Beschreibung der Figuren

Figur 1 zeigt ein Prozessschema von bevorzugten Ausführungsformen des erfindungsgemäßen Verfahrens zur Umsetzung von C2- oder C4-Quellen zu Methacrylsäure bzw. Alkylmethacrylaten.

Figur 2 zeigt den zeitlichen Verlauf der di-MAL-Konzentrationen in MAL in Abhängigkeit von Lagerzeit und -temperatur.

Figur 3 zeigt einen vergrößerten Ausschnitt aus Figur 2.

Figur 4 zeigt die Zusammenfassung der in den Beispielen 2 bis 4 beobachteten Ölbadtemperaturen bei Oxidation von Methacrolein (c[di-$MAL_{frisch}$] = 35 ppm), das vor Versuchsbeginn bei der Temperatur $T_L$ für die Dauer $t_L$ gelagert wurde.

Apparate

A Erster Oxidationsreaktor
B Zweiter Oxidationsreaktor
C Separationsvorrichtung
D Zwischenbehälter
E Verdampfer
F Veresterungsreaktor
G Direkte oxidative Veresterung
H Propionaldehyd-Synthesereaktor
I Propionaldehyd-Katalysatorabtrennung
J Methacrolein-Synthesereaktor
K Methacrolein-Katalysatorabtrennung
L Mischpunkt 1
M Mischpunkt 2

Ströme

1 Methacrolein-haltiges Prozeßgas aus dem ersten Oxidationsreaktor
2 Methacrylsäure-haltiges Prozeßgas aus dem zweiten Oxidationsreaktor
3a Überwiegend Methacrylsäure enthaltener Strom aus der Separationsvorrichtung C
3b Überwiegend Methacrolein enthaltender Strom aus der Separationsvorrichtung C
4 Gekühlter, überwiegend Methacrolein enthaltender Strom im Zustrom zu Verdampfer E
5 Methacrolein enthaltender Gasstrom
6 Sauerstoff und Wasserdampf enthaltendes Gasgemisch
7 Methacrolein-haltiger Prozeßgas-Feed zum zweiten Oxidationsreaktor
8 Flüssiger überwiegend Propionaldehyd enthaltender Prozeßstrom
9 Flüssiger überwiegend Hydroformylierungskatalysator enthaltender Prozeßstrom
10 Aufgereinigter flüssiger Propionaldehyd enthaltender Strom
11 Flüssiger überwiegend Methacrolein enthaltender Prozeßstrom
12 Flüssiger überwiegend das Mannich-Katalysatorsystem enthaltender Prozeßstrom
13 Aufgereinigter flüssiger Methacrolein enthaltender Strom
14 Gekühlter, überwiegend Methacrolein enthaltender Strom im Zustrom zu Reaktor G

f1 $O_2$-haltiges Gas im Zustrom zu Reaktor A
f2 C4-Quelle im Zustrom zu Reaktor A

| | |
|---|---|
| f3 | $O_2$-haltiges Gas zur Anmischung des Sauerstoff und Wasserdampf enthaltendes Gasgemisch 6 |
| f4 | Wasserdampf zur Anmischung des Sauerstoff und Wasserdampf enthaltendes Gasgemisch 6 |
| f5 | Synthesegasstrom im Zustrom zu Reaktor H |
| f6 | C2-Quelle im Zustrom zu Reaktor H |
| f7 | Formalin enthaltender Strom im Zustrom zu Reaktor J |
| f8 | Alkohol enthaltender Strom im Zustrom zu Reaktor F |
| f9 | Alkohol enthaltender Strom im Zustrom zu Reaktor G |
| f10 | $O_2$-haltiges Gas im Zustrom zu Reaktor G |

| | |
|---|---|
| p1 | Optional aufgereinigter Methacrylsäure-Produktstrom aus Reaktor C |
| p2 | Optional aufgereinigter Alkylmethacrylat-Produktstrom aus Reaktor F |
| p3 | Optional aufgereinigter Alkylmethacrylat-Produktstrom aus Reaktor G |

Sonstige Bezugszeichen

| | |
|---|---|
| t(L) | Lagerzeit im Zwischenbehälter D |
| T(L) | Lagertemperatur im Zwischenbehälter D |
| GC | Gaschromatographie |
| MAL | Methacrolein |
| di-MAL | Cyclisches Methacrolein-Dimer |
| ppm | Parts per million |
| $E_A$ | Aktivierungsenergie [kJ/mol] |
| A | Stoßfaktor [ppm/d] |
| c(i) | Konzentration der Komponente i |
| GHSV | Gas Hourly Space Velocity |
| X(i) | Umsatz der Komponente i [%] |
| S(i) | Selektivität der Komponente i [%] |
| Y(i) | Ausbeute der Komponente i [%] |
| MAS | Methacrylsäure |
| MMA | Methylmethacrylat |
| T(Oil) | Ölbad-Temperatur |
| T(35 cm) | Reaktortemperatur innerhalb des Katalysatorbetts in 35 cm Abstand zum Reaktoreingang |
| T(45 cm) | Reaktortemperatur innerhalb des Katalysatorbetts in 45 cm Abstand zum Reaktoreingang |
| SAPT | Self accelerating polymerization temperature [°C] |
| Sdp. | Siedepunkt [°C] |
| DOE | Direkte Oxidative Veresterung |
| PMMA | Poly(methylmethacrylat) |
| IBEN | Isobuten |
| TBA | Tert.-Butylalkohol |
| MTBE | Methyl-tert.-butylether |

Beschreibung der Abbildungen

Zu den Abbildungen sei angemerkt, dass weitere dem Fachmann bekannte Komponenten zusätzlich zur Durchführung des erfindungsgemäßen Verfahrens enthalten sein können. So weist beispielsweise in der Regel jede der aufgeführten Kolonnen einen Kondensator auf. Auch sei angemerkt, dass in den Zeichnungen nicht jede bevorzugte Ausführungsform berücksichtigt ist. Die Position der Zuleitungen gibt zudem nicht deren reale Lage an, sondern verdeutlicht nur die topologische Anordnung der entsprechenden Prozessschritte.

Figur 1 zeigt das allgemeine Prozessschema der Umsetzung von C2- oder C4-Quellen mit einem Sauerstoff und Wasserdampf enthaltenden Gas zu Methacrylsäure oder Alkylmethacrylaten.

Dabei wird in einer Variante eine C4-Quelle **f2** zusammen mit einem Sauerstoff enthaltenden Gas **f1** in einem Reaktor **A** zu einem Methacrolein enthaltenden Prozessgas **1** umgesetzt, das nach Hinzumischung eines weiteren Methacrolein-haltigen Prozessgases **5** sowie optional eines aus einem Sauerstoff enthaltenden Strom **f3** und einem Wasserdampf enthaltenden Strom **f4** hervorgehenden Gasstroms **6** einen Methacrolein enthaltenden Prozessstrom **7** ergibt, der einem Reaktor **B** zugeleitet wird. In Reaktor **B** wird der Methacrolein enthaltende Prozessstrom **7** in einen Methacrylsäure enthaltenden Prozessstrom **2** umgewandelt, der in der nachfolgenden Separationsvorrichtung **C** in einen bevorzugt

Methacrylsäure enthaltenden Strom **3a** und einen bevorzugt Methacrolein enthaltenden Prozessstrom **3b** aufgetrennt wird. Aus dem bevorzugt Methacrylsäure enthaltenden Strom **3a** kann nach optionalen Reinigungsschritten direkt ein Methacrylsäure enthaltender Produktstrom **p1** gewonnen werden. Alternativ kann der bevorzugt Methacrylsäure enthaltender Prozessstrom **3a** zusammen mit einem Alkohol enthaltenden Strom **f8** in einem Reaktor **F** zu einem Alkylmethacrylate enthaltenden Produktstrom **p2** umgesetzt werden. Der bevorzugt Methacrolein enthaltende Prozessstrom **3b** wird in flüssiger Form in einem Zwischenbehälter **D** gekühlt gelagert und als gekühlter Prozessstrom **4** einem Verdampfer **E** zugeleitet, wo er in das zuvor beschriebene Methacrolein-haltige Prozessgas **5** überführt wird, das wie zuvor dargelegt wieder vor den Eingang von Reaktor **B** geleitet wird.

Optional kann der flüssige, bevorzugt Methacrolein enthaltende Prozessstrom **14** gemeinsam mit einer Sauerstoff enthaltenden Gasmischung **f10** und einem Alkohol enthaltenden Strom **f9** in einem Reaktor **G** in einer Direkten Oxidativen VeresterungsReaktion direkt zu einem Alkylmethacrylate enthaltenden Produktstrom **p3** umgesetzt werden. In einer weiteren, alternativen Variante wird eine C2-Quelle **f6** zusammen mit Synthesegas **f5** und einem Katalysator in einem Hydroformylierungsreaktor **H** zu einem Propionaldehyd enthaltenden Prozessstrom **8** umgewandelt, der in einer Separationsvorrichtung **I** in einen bevorzugt Katalysator-haltigen Prozessstrom **9** und einen Katalysator-armen Propionaldehyd enthaltenden Strom **10** aufgetrennt wird. Während der bevorzugt Katalysator-haltige Prozesstrom **9** wieder dem Hydroformylierungsreaktor **H** zugeleitet wird, wird der Katalysator-arme Propionaldehyd enthaltende Strom **10** gemeinsam mit einem Formalin enthaltenden Strom **f7** und einem weiteren Katalysator im Reaktor **J** zu einem Methacrolein-haltigen Strom **11** umgewandelt, der in der nachfolgenden Separationsvorrichtung **K** in einen bevorzugt Katalysator-haltigen Prozessstrom **12** sowie einen bevorzugt Methacrolein enthaltenden flüssigen Prozessstrom **13** aufgetrennt wird. Der bevorzugt Methacrolein enthaltende flüssige Prozessstrom **13** kann in dieser Prozessvariante unmittelbar dem bereits beschriebenen Verdampfer **E** zugeführt werden, wo er zusammen mit dem gekühlten, bevorzugt Methacrolein enthaltenden Prozessstrom **4** in das Methacrolein-haltiges Prozessgas **5** überführt wird. Alternativ kann der bevorzugt Methacrolein enthaltende flüssige Prozessstrom **13** zunächst zusammen mit dem bereits beschriebenen bevorzugt Methacrolein enthaltenden Prozessstrom **3b** in dem ebenfalls bereits beschriebenen Zwischenbehälter **D** gekühlt gelagert werden unter Bildung des gekühlten Prozessstroms **4**. Das in beiden Alternativen erhältliche Methacrolein-haltige Prozessgas **5** kann wie zuvor beschrieben in Methacrylsäure oder Alkylmethacrylate umgewandelt werden.

## Beispiele

### Beispiel 1 (Bildungskinetik von di-MAL aus MAL)

Für die Untersuchung der Bildungskinetik von di-MAL aus MAL wurde MAL bei unterschiedlichen Temperaturen für 46 Tage gelagert und die di-MAL-Konzentration kontinuierlich mittels GC überwacht. Das für die Versuche eingesetzte MAL wurde vorab destilliert, um eine möglichst geringe di-MAL-Startkonzentration zu erreichen (hier: 35 ppm). Aus den in Tabelle 2 und Fig. 2 sowie Fig. 3 zusammengefassten ermittelten di-MAL-Konzentrationen lässt sich eine di-MAL-Bildungskinetik pseudo-nullter Ordnung mit folgendem Bildungsgesetz ableiten ($E_A$ = 90,25 kJ/mol, A = 4,57 * $10^{18}$ ppm/d):

$$c(t) = c_0 + \left(A * e^{\frac{-E_A}{R*T}}\right) * t$$

Bei einer Lagertemperatur von -5 °C kann mit einer Bildung von 12 ppm MAL pro Tag gerechnet werden. Bei 25 °C werden dagegen schon knapp 700 ppm MAL pro Tag gebildet; bei 50 °C 1,2 %/d.

*Tabelle 2: Konzentration von di-MAL in MAL in Abhängigkeit von der Lagertemperatur T(L) und -dauer t(L)*

| **Probe** | **T(L) [d]** | **c(di-Mal) (ppm)** | | |
|---|---|---|---|---|
| | | **T(L) = -19.1 °C** | **T(L) = 4.8 °C** | **T(L) = 23.5 °C** |
| **0** | 0 | 35,35 | 35,35 | 40 |
| **1** | 1 | 38,47 | 56,79 | 900 |
| **2** | 4 | 41,64 | 138,06 | 3400 |
| **3** | 5 | 53,95 | 183,27 | 4500 |
| **4** | 7 | 64,81 | - | 6400 |

(fortgesetzt)

| | | T(L) = -19.1 °C | T(L) = 4.8 °C | T(L) = 23.5 °C |
|---|---|---|---|---|
| **5** | 11 | 58,2 | 300,14 | 11400 |
| **6** | 13 | 58,05 | 370,8 | 11900 |
| **7** | 15 | 60,69 | 438,89 | 12900 |
| **8** | 18 | 64,8 | 484,05 | 14400 |
| **9** | 20 | 70,25 | 554,75 | 17600 |
| **10** | 26 | 104,33 | 749,8 | 21600 |
| **11** | 32 | 96,35 | 865,74 | 27400 |
| **12** | 46 | 106,27 | 1239,89 | 40500 |

**Vergleichsbeispiel 1 (Gasphasenoxidation von MAL; c(di-MAL) = 10.000 ppm)**

Die Gasphasenoxidation von MAL zu MAS wurde in einem kontinuierlich betriebenen Rohrreaktor untersucht. Zur Kontrolle der Reaktionstemperatur ist der Reaktor mit 2 Thermoelementen im Abstand von 35 cm bzw. 45 cm vom Reaktoreingang ausgestattet. Für den Versuch wird Methacrolein mit einem di-MAL-Gehalt von 35 ppm zunächst für 10 Tage bei 25 °C gelagert, wonach es einen di-MAL-Gehalt von 10.000 ppm aufweist. Das MAL wird in einem Verdampfer bei 160 °C mithilfe eines Gasstroms aus Luft und Stickstoff in die Gasphase überführt. Die nach weiterer Zugabe von Luft und Stickstoff resultierende Gasmischung (MAL / $O_2$ / $H_2O$ / $N_2$ = 1 : 2.5 : 4.5 : 22.5) wird mit einer GHSV von 1070 $h^{-1}$ über einen Molybdän-Bismut-Mischoxid-Katalysator geleitet und dabei die Temperatur des Ölbads so eingestellt, dass ein MAL-Umsatz X(MAL) von 65,8 % resultiert. Der MAL-Umsatz wird durch Vergleich der der mittels GC ermittelten Gaszusammensetzungen am Eintritt und Austritt des Reaktors bestimmt. Nachdem sich der Umsatz nach 12 h Betriebszeit stabilisiert hat, wird die Ölbad-Temperatur so weit angehoben, dass ein MAL-Umsatz X(MAL) von 68,8 % erreicht wird. Auf gleiche Weise werden durch weitere Erhöhung der Ölbad-Temperatur noch Umsätze von 74,9 und 75,3 % eingestellt. Die benötigten Ölbad-Temperaturen (T[Oil]) sowie die beobachteten Methacrylsäure-Selektivitäten (S[MAS]) und Temperaturen innerhalb des Katalysatorbetts in 35 cm bzw. 45 cm Abstand zum Reaktoreingang sind in Tabelle 3 zusammengefasst.

*Tabelle 3: Temperaturen T, Umsätze X(MAL) und Methacrylsäure-Selektivitäten S(MAS) bei der Gasphasenoxidation von Methacrolein (di-MAL-Gehalt 10.000 ppm)*

| Beisp iel | X(MAL) / % | S(MAS) / % | T(Oil) /°C | T(35 cm) / °C | T (45 cm) / °C |
|---|---|---|---|---|---|
| **2-1** | 65,8 | 89,7 | 288,5 | 307,1 | 308 |
| **2-2** | 68,8 | 89,3 | 289,8 | 312 | 311,2 |
| **2-3** | 74,9 | 88,6 | 293,7 | 323,2 | 319,7 |
| **2-4** | 75,3 | 89 | 293,7 | 322 | 317,7 |

**Beispiel 2 (Gasphasenoxidation von MAL; c(di-MAL) = 5000 ppm; Effekt der geringeren Lagerzeit)**

Die Gasphasenoxidation von MAL zu MAS wurde analog Vergleichsbeispiel 1 untersucht. Für den Versuch wird Methacrolein mit einem di-MAL-Gehalt von 35 ppm zunächst für 5 Tage bei 25 °C gelagert, wonach es einen di-MAL-Gehalt von 5000 ppm aufweist. Die nach MAL-Verdampfung und Zumischung von Stickstoff und Luft resultierende Gasmischung wird wie in Vergleichsbeispiel 1 beschrieben über den Katalysator geleitet und dabei die Temperatur des Ölbads so eingestellt, dass ein MAL-Umsatz von 66,3 % resultiert. Nachdem sich der Umsatz nach 12 h Betriebszeit stabilisiert hat, wird die Ölbad-Temperatur so weit angehoben, dass ein MAL-Umsatz X(MAL) von 67,8 % erreicht wird. Auf gleiche Weise werden durch weitere Erhöhung der Ölbad-Temperatur noch Umsätze von 70,1 und 75,4 % eingestellt. Die benötigten Ölbad-Temperaturen (T[Oil]) sowie die beobachteten Methacrylsäure-Selektivitäten (S[MAA]) und Temperaturen innerhalb des Katalysatorbetts in 35 cm bzw. 45 cm Abstand zum Reaktoreingang sind in Tabelle 4 zusammengefasst.

*Tabelle 4: Temperaturen, Umsätze und Methacrylsäure-Selektivitäten bei der Gasphasenoxidation von Methacrolein (Di-MAL-Gehalt 5.000 ppm)*

| Beispiel | X(MAL) / % | S(MAA) / % | T(Öl) /°C | T(35 cm) / °C | T (45 cm) / °C |
|---|---|---|---|---|---|
| **3-1** | 66,3 | 88,5 | 287,4 | 311,4 | 317,1 |
| **3-2** | 67,8 | 87,8 | 287,4 | 311,4 | 317,1 |
| **3-3** | 70,1 | 87,7 | 289 | 315,6 | 320,5 |
| **3-4** | 75,4 | 87,6 | 291,7 | 321,6 | 319 |

**Beispiel 3 (Gasphasenoxidation von MAL; Di-MAL-Gehalt 300 ppm, Effekt der geringeren Lagertemperatur)**

Die Gasphasenoxidation von MAL zu MAS wurde analog Vergleichsbeispiel 1 untersucht. Für den Versuch wird Methacrolein mit einem di-MAL-Gehalt von 35 ppm zunächst für 10 Tage bei 5 °C gelagert, wonach es einen di-MAL-Gehalt von 300 ppm aufweist. Die nach MAL-Verdampfung und Zumischung von Stickstoff und Luft resultierende Gasmischung wird wie in Vergleichsbeispiel 1 beschrieben über den Katalysator geleitet und dabei die Temperatur des Ölbads so eingestellt, dass ein MAL-Umsatz von 64,0 % resultiert. Nachdem sich der Umsatz nach 12 h Betriebszeit stabilisiert hat, wird die Ölbad-Temperatur so weit angehoben, dass ein MAL-Umsatz von 65,5 % erreicht wird. Auf gleiche Weise werden durch weitere Erhöhung der Ölbad-Temperatur noch Umsätze von 69,0 %, 71,5 %, 74,3 %, 74,9 % und 76,4 % eingestellt. Die benötigten Ölbad-Temperaturen (T[Öl]) sowie die beobachteten Methacrylsäure-Selektivitäten (S[MAS]) und Temperaturen innerhalb des Katalysatorbetts in 35 cm bzw. 45 cm Abstand zum Reaktoreingang sind in Tabelle 5 zusammengefasst.

*Tabelle 5: Temperaturen, Umsätze und Methacrylsäure-Selektivitäten bei der Gasphasenoxidation von Methacrolein (di-MAL-Gehalt 300 ppm)*

| Beisp iel | X(MAL) / % | S(MAS) / % | T(Oil) /°C | T(35 cm) / °C | T (45 cm) / °C |
|---|---|---|---|---|---|
| **4-1** | 64 | 89,6 | 285,1 | 303,4 | 318,4 |
| **4-2** | 65,5 | 88,5 | 285,2 | 308,6 | 312,7 |
| **4-3** | 69 | 86,8 | 287,4 | 308,4 | 323,3 |
| **4-4** | 71,5 | 87,3 | 287,4 | 316,3 | 318,5 |
| **4-5** | 74,3 | 84,2 | 290,4 | 321,4 | 324,4 |
| **4-6** | 74,9 | 83 | 290,3 | 314,3 | 336,6 |
| **4-7** | 76,4 | 88,6 | 290,9 | 320,7 | 313,4 |

Die in Vergleichsbeispiel 1 sowie den Beispielen 2 und 3 beobachteten Ölbadtemperaturen sind in Fig. 4 zusammengefasst. Es wird deutlich, dass für den gesamten untersuchten Umsatzbereich jenes MAL die höchste Ölbadtemperatur erfordert, das bei der höchsten Temperatur (25 °C) für die längste Zeit (10 d) gelagert wurde. Durch Halbierung der Lagerzeit kann die Ölbadtemperatur um durchschnittlich 2 K gesenkt werden. Durch Reduzierung der Lagertemperatur von 25 °C auf 5 °C bei gleicher Lagerzeit kann die Ölbadtemperatur sogar um durchschnittlich 3 K gesenkt werden. Eingedenk des negativen Einflusses der Badtemperatur auf die Lebenszeit eines Methacrolein-Oxidationskatalysators kann somit eine Lebenszeitverlängerung des Katalysators dadurch bewirkt werden, dass zwischengespeichertes MAL bei möglichst geringen Temperaturen für möglichst geringe Zeitspannen gelagert wird.

**Vergleichsbeispiel 2 (Direkte Oxidative Veresterung von MAL in der Flüssigphase im Batch-Test; c(di-MAL) = 10.000 ppm;)**

Für den Versuch wird Methacrolein mit einem di-MAL-Gehalt von 35 ppm zunächst für 10 Tage bei 25 °C gelagert, wonach es einen di-MAL-Gehalt von 10.000 ppm aufweist. Das so vorbehandelte MAL (1,2 g) wird zusammen mit einem AuCoO/$SiO_2$-$Al_2O_3$-MgO-Katalysator (384 mg) und Methanol (9,48 g) in einem 140 mL Stahlautoklaven mit einem Magnetrührer suspendiert. Der pH-Wert des MAL wurde mit 1% NaOH in MeOH zunächst auf 7,0 eingestellt, und das MAL wurde mit 100 ppm Tempol stabilisiert. Auf den Autoklaven wurden 30 bar Überdruck gepresst mit einer Gasmischung aus 7% $O_2$ in $N_2$. Das Explosionslimit der Mischung liegt 8 Vol% Sauerstoff. Der Autoklav wurde für 2

Stunden auf 80 °C erhitzt, abgekühlt, entgast und die Suspension filtriert. Das Filtrat wurde mittels GC analysiert. Der Umsatz an MAL war 64,0 %, die Selektivität zu MMA 93,7 % und die Raum-Zeit Ausbeute war 10.6 mol MMA / kg Katalysator pro Stunde. Das Filtrat enthielt ferner 500 ppm di-MAL und 10.450 ppm di-MAL-Ester. Es zeigt sich, dass das im Feed enthaltene di-MAL zu 95 % zum di-MAL-Methylester oxidativ verestert wird und zu rund 5 % unverändert aus der Reaktion hervorgeht.

**Beispiel 4 (Direkte Oxidative Veresterung von MAL in der Flüssigphase im Batch-Test; c(di-MAL) = 300 ppm; Effekt der geringen Lagertemperatur)**

Die direkt oxidative Veresterung von MAL zu MMA wurde analog Vergleichsbeispiel 2 untersucht. Für den Versuch wird Methacrolein mit einem di-MAL-Gehalt von 35 ppm zunächst für 10 Tage bei 5 °C gelagert, wonach es einen di-MAL-Gehalt von 300 ppm aufweist. Das so vorbehandelte MAL (1,2 g) wird zusammen mit einem Au-CoO/$SiO_2$-$Al_2O_3$-MgO-Katalysator (384 mg) und Methanol (9,48 g) in einem 140 mL Stahlautoklaven mit einem Magnetrührer suspendiert. Der pH-Wert des MAL wurde mit 1% NaOH in MeOH zunächst auf 7,0 eingestellt, und das MAL wurde mit 100 ppm Tempol stabilisiert. Auf den Autoklaven wurden 30 bar Überdruck gepresst mit einer Gasmischung aus 7% $O_2$ in $N_2$. Das Explosionslimit der Mischung liegt 8 Vol% Sauerstoff. Der Autoklav wurde für 2 Stunden auf 80 °C erhitzt, abgekühlt, entgast und die Suspension filtriert. Das Filtrat wurde mittels GC analysiert. Der Umsatz an MAL war 67,0 %, die Selektivität zu MMA 93,7 % und die Raum-Zeit Ausbeute war 11,1 mol MMA / kg Katalysator pro Stunde. Das Filtrat enthielt ferner 15 ppm di-MAL und 315 ppm Di-MAL-Ester. Es zeigt sich, dass das im Feed enthaltene Di-MAL zu 95 % zum Di-MAL-Methylester oxidativ verestert wird und zu rund 5 % unverändert aus der Reaktion hervorgeht. Im Vergleich zu Beispiel 5 zeigt sich zusätzlich, dass durch den infolge der geringen Lagertemperatur verminderten di-MAL-Gehalt im Feed die Raum-ZeitAusbeute der Zielreaktion erhöht werden konnte.

## Patentansprüche

1. Verfahren zur Herstellung von Methacrylsäure und/oder von Alkylmethacrylaten ausgehend von einer C2-Quelle **f6** oder einer C4-Quelle **f2** in einem ersten Reaktor unter Entstehung einer Methacrolein-haltigen Fraktion und einer weiteren anschließenden Umsetzung einer auf der ersten Fraktion basierenden zweiten Methacrolein-haltigen Fraktion in mindestens einem zweiten Reaktor, **dadurch gekennzeichnet, dass** die Methacrolein-haltige Fraktion in flüssiger Form in einem Zwischenbehälter **D** auf eine Lagertemperatur zwischen -30 °C und 50 °C gekühlt mit einer Verweilzeit kleiner 48 h gelagert wird und von dort in einen Verdampfer **E** oder in flüssiger Form in den zweiten Reaktor, bei dem es sich um einen Reaktor **G** für eine oxidative Veresterung handelt, geleitet wird.

2. Verfahren gemäß Anspruch 1, **dadurch gekennzeichnet, dass** ausgehend von einer C4-Quelle **f2** in dem ersten Reaktor **A** ein Methacrolein-haltiges Prozessgas **1** als Methacrolein-haltige Fraktion gebildet wird.

3. Verfahren gemäß Anspruch 1, **dadurch gekennzeichnet, dass** ausgehend von einer C2-Quelle **f6** in einem Reaktor **H** unter Entstehung eines Propionaldehyd-haltigen Stroms **8**, der mittels mindestens eine Destillation umfassenden Reinigungsschritts **I** aufgearbeitet wird, ein Propionaldehyd enthaltender Strom **10** gebildet wird, der wiederum in einem nachfolgenden ersten Reaktor **J** und einem weiteren nachfolgenden Reinigungsschritt **K** zu einem Methacrolein-haltigen Strom **13** umgewandelt wird und dieser Methacrolein-haltigen Strom **13** in den Zwischenbehälter **D** geleitet wird.

4. Verfahren gemäß Anspruch 3, **dadurch gekennzeichnet, dass** ein flüssiger Methacrolein-haltigen Strom **4** aus dem Zwischenbehälter **D** in einem Verdampfer **E** geleitet wird und dort unter Entstehung eines Methacrolein-haltigen Prozessgases **5** verdampft wird.

5. Verfahren gemäß Anspruch 2 oder 4, **dadurch gekennzeichnet, dass** ein Methacrolein-haltiges Prozessgas **1** oder **5** zusammen mit einem Sauerstoff und Wasserdampf enthaltenden Gasgemisch **6**, optional vorvermischt als Gasgemisch **7** in den zweiten Reaktor, bei dem es sich um einen Oxidationsreaktor **B** handelt, geleitet wird, wobei in diesem zweiten Reaktor **B** ein Methacrylsäure-haltiges Prozessgas **2** gebildet wird, wobei

    a. das Prozessgas **2** in einer Separationsvorrichtung **C** in einen überwiegend Methacrylsäure enthaltenden Strom **3a** und einen überwiegend Methacrolein enthaltenden Strom **3b** aufgetrennt wird,
    b. die in Schritt a beschriebene Separationsvorrichtung jeweils mindestens einen der Schritte Quenchen, Kristallisation und Destillation umfasst,
    c. der überwiegend Methacrolein-haltige Strom **3b** am Kopf einer fraktionierenden Destillationskolonne kondensiert und der kondensierte Methacrolein-haltige Strom **3b** danach in den Zwischenbehälter **D** geleitet wird und d. aus Zwischenbehälter **D** ein thermostatisierter Methacrolein-haltiger Strom **4** anschließend in den bzw. einen Verdampfer **E** geleitet wird, um dort Strom **4** in einen Methacrolein-haltigen Gasstrom **5** zu überführen, der zusammen mit Gasgemisch **6**, optional gemischt zu einem Gasstrom **7**, in Reaktor **B** geleitet wird.

**6.** Verfahren gemäß Anspruch 5, **dadurch gekennzeichnet, dass** der überwiegend Methacrylsäure enthaltende Strom **3a** destillativ und/oder extraktiv aufgereinigt wird und optional in einem weiteren Schritt mit einem Alkohol in einem Reaktor **F** sauer katalysiert zu einem Alkylmethacrylat umgesetzt wird.

**7.** Verfahren gemäß mindestens einem der Ansprüche 1 bis 3, **dadurch gekennzeichnet, dass** ein Methacrolein-haltiger Strom **14** in flüssiger Form aus Zwischenbehälter **D** entnommen wird und in einem Reaktor **G** in Gegenwart eines Sauerstoff-haltigen Gases **f10**, eines Katalysators und eines Alkylalkohols **f9** in flüssiger Phase in einer direkten oxidativen Veresterung zu einem Alkylmethacrylat-haltigen Stoffgemisch umgesetzt wird.

**8.** Verfahren gemäß Anspruch 2 und 5, **dadurch gekennzeichnet, dass** im Prozessgas 1 der Isobuten-Gehalt einen Wert von 2000 Vol-ppm nicht übersteigt und Aufarbeitung, Thermostatisierung und erneute Verdampfung des aus dem Methacrolein-haltigen Prozessgases **2** hervorgehenden Methacrolein-haltigen Stroms **4** einen Methacrolein-haltigen Strom **5** ergibt, der, bezogen auf Methacrolein, 0,2 bis 25 Gew% weitere carbonylische $C_1$-$C_4$-Kohlenwasserstoffe enthält und einen Gehalt an Methacrolein-Dimeren (di-MAL) von kleiner 1 Gew% aufweist.

**9.** Verfahren gemäß Anspruch 5 oder 6, **dadurch gekennzeichnet, dass** der nach Aufarbeitung und Abtrennung des überwiegend Methacrylsäure enthaltenden Stroms **3a** erhaltene überwiegend Methacrolein-haltige Stoffstrom **3b** derart kondensiert und vorgelagert wird, dass die Konzentration von Methacrolein-Dimeren im flüssigen Feedstrom **4** zum Verdampfer **E** bzw. zu Reaktor **G** einen Gehalt von 1 Gew% nicht überschreitet.

**10.** Verfahren gemäß Anspruch 5 oder 6, **dadurch gekennzeichnet, dass** die Kondensationstemperatur des Methacrolein-haltigen Stroms **2** höher ist als die Lagertemperatur des Methacrolein-haltigen Stroms im Zwischenbehälter **D**.

**11.** Verfahren gemäß mindestens einem der Ansprüche 1 bis 10, **dadurch gekennzeichnet, dass** die Verweilzeit des bevorzugt Methacrolein enthaltenden Stroms **3b** oder **13** im Zwischenbehälters **D** kleiner als 12 h ist und die Lagertemperatur zwischen -20 °C und 30 °C beträgt.

**12.** Verfahren gemäß Anspruch 11, **dadurch gekennzeichnet, dass** die Verweilzeit im Zwischenbehälters **D** kleiner als 6 h ist und die Lagertemperatur zwischen -10 °C und 20 °C beträgt.

Fig. 1

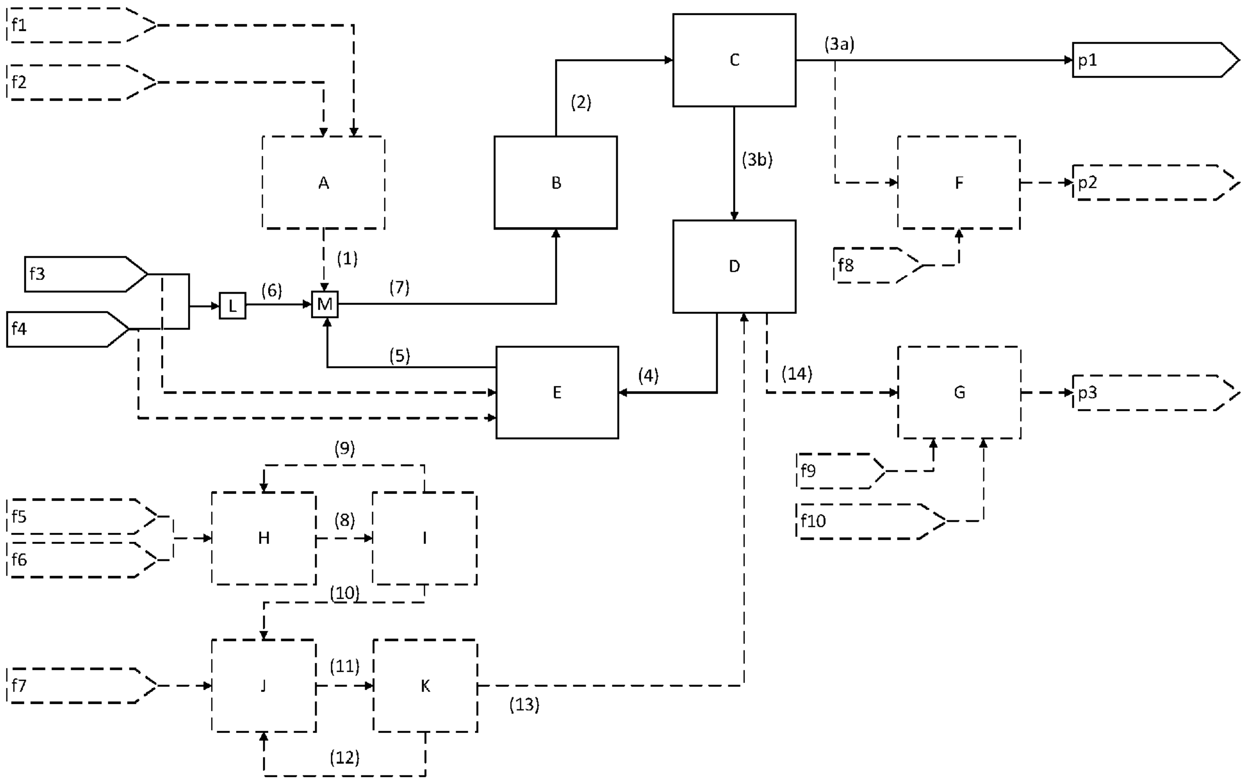
f1
f2
A
(1)
f3
f4
L
(6)
M
(7)
(5)
B
(2)
C
(3a)
p1
(3b)
D
(4)
E
F
p2
f8
(14)
G
p3
f9
f10
(9)
f5
f6
H
(8)
I
(10)
f7
J
(11)
K
(13)
(12)

Fig. 2

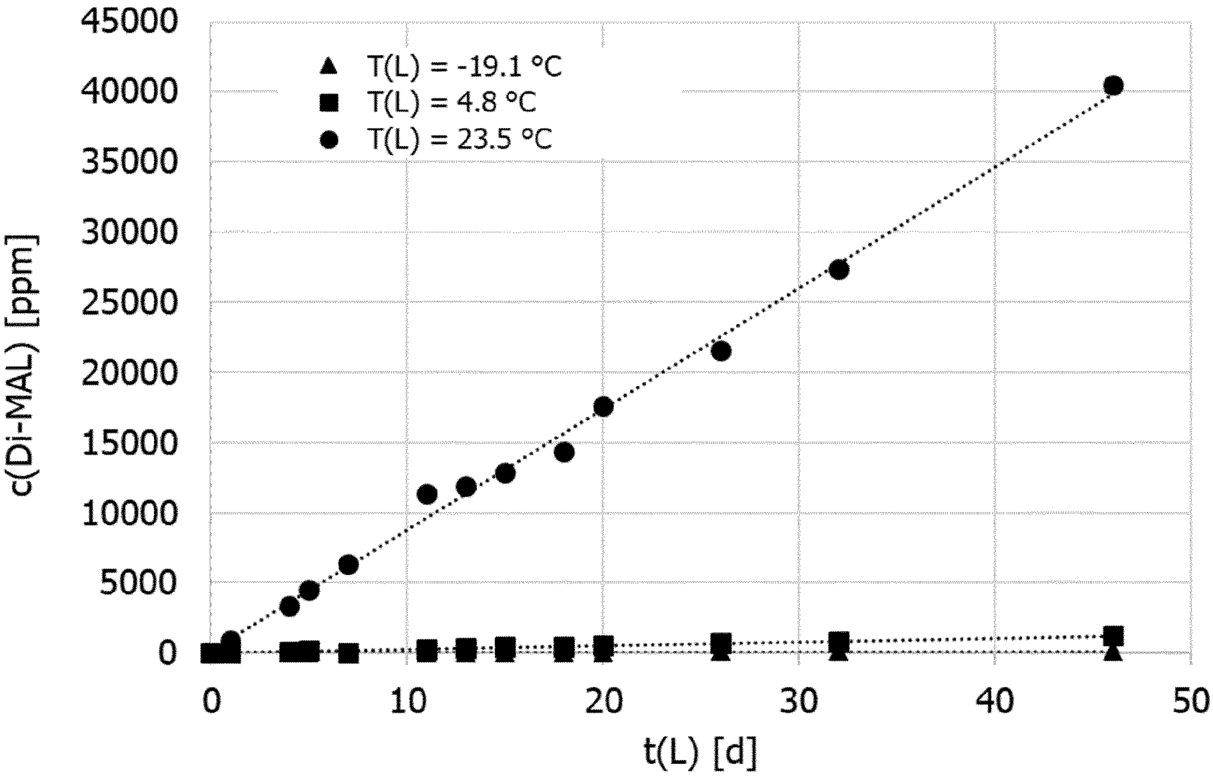
45000
40000
35000
30000
25000
20000
15000
10000
5000
0
c(Di-MAL) [ppm]
T(L) = -19.1 °C
T(L) = 4.8 °C
T(L) = 23.5 °C
0
10
20
30
40
50
t(L) [d]

Fig. 3

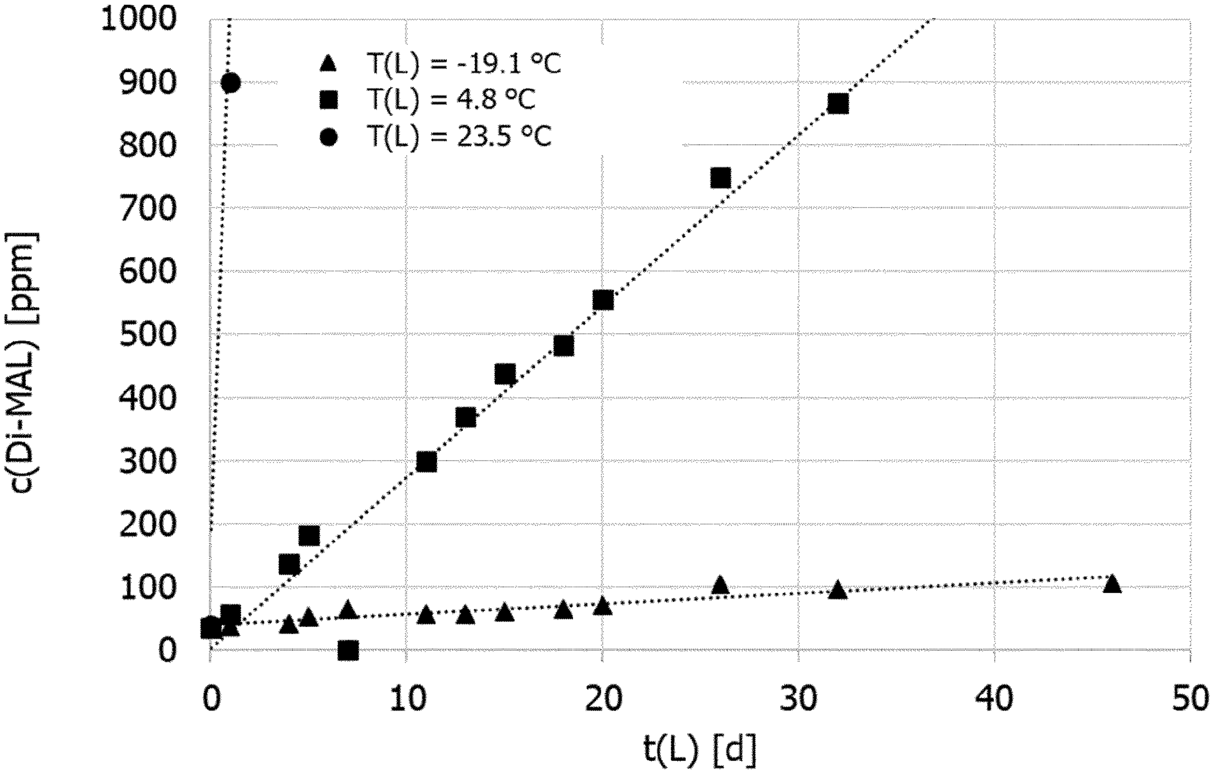

1000
900
800
700
600
500
400
300
200
100
0
c(Di-MAL) [ppm]
0
10
20
30
40
50
t(L) [d]
T(L) = -19.1 °C
T(L) = 4.8 °C
T(L) = 23.5 °C

Fig. 4

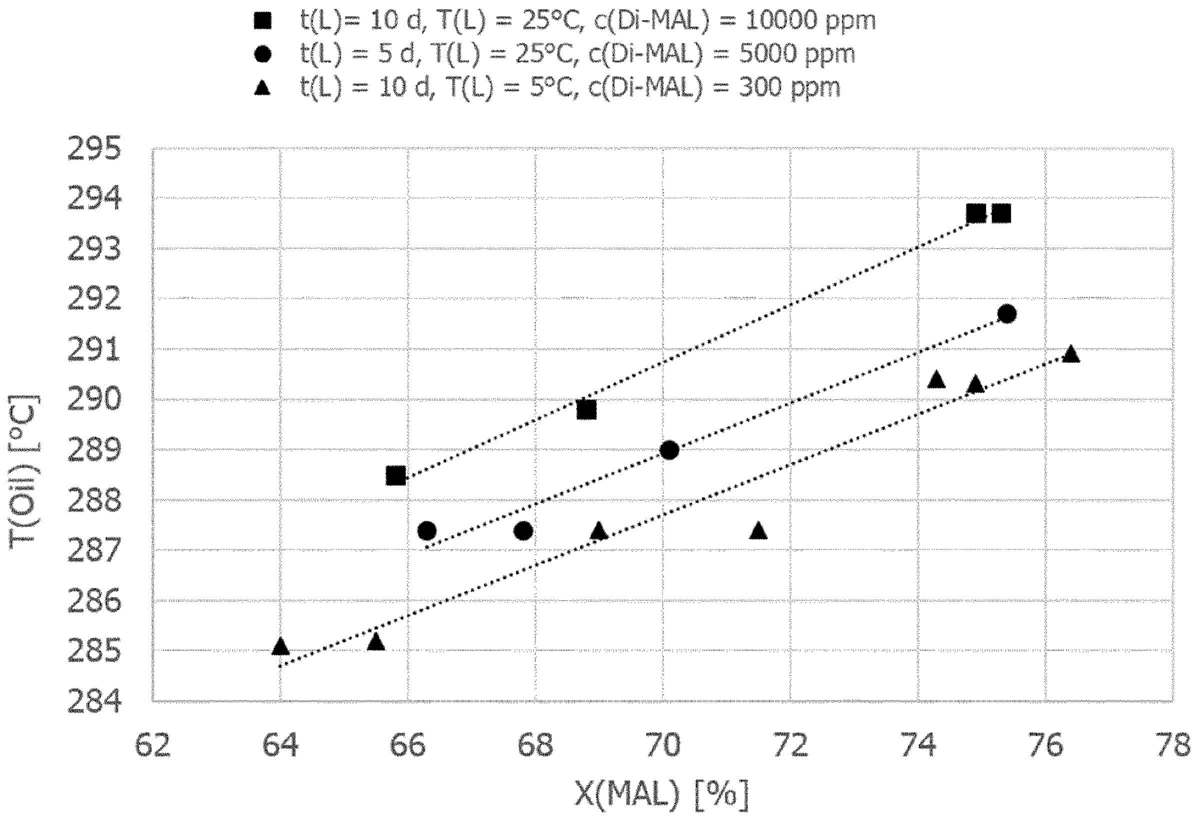
t(L)= 10 d, T(L) = 25°C, c(Di-MAL) = 10000 ppm
t(L) = 5 d, T(L) = 25°C, c(Di-MAL) = 5000 ppm
t(L) = 10 d, T(L) = 5°C, c(Di-MAL) = 300 ppm
295
294
293
292
291
290
289
288
287
286
285
284
T(Oil) [°C]
62
64
66
68
70
72
74
76
78
X(MAL) [%]

Europäisches Patentamt
European Patent Office
Office européen des brevets

# EUROPÄISCHER RECHERCHENBERICHT

Nummer der Anmeldung
EP 20 18 8528

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A,D | WO 2014/170223 A1 (EVONIK INDUSTRIES AG [DE]; KRILL STEFFEN [DE] ET AL.) 23. Oktober 2014 (2014-10-23) * das ganze Dokument * ----- | 1-12 | INV. C07C67/39 C07C69/54 |
| A,D | EP 0 194 620 A2 (BASF AG [DE]) 17. September 1986 (1986-09-17) * das ganze Dokument * ----- | 1-12 | |
| A,D | EP 3 450 422 A1 (EVONIK ROEHM GMBH [DE]) 6. März 2019 (2019-03-06) * das ganze Dokument * ----- | 1-12 | |
| A | WO 2017/046110 A1 (EVONIK RÖHM GMBH [DE]) 23. März 2017 (2017-03-23) * das ganze Dokument * ----- | 1-12 | |

RECHERCHIERTE SACHGEBIETE (IPC)
C07C

Der vorliegende Recherchenbericht wurde für alle Patentansprüche erstellt

2

| Recherchenort | Abschlußdatum der Recherche | Prüfer |
|---|---|---|
| Den Haag | 8. März 2021 | Fritz, Martin |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR.

EP 20 18 8528

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

08-03-2021

| Im Recherchenbericht angeführtes Patentdokument | Datum der Veröffentlichung | Mitglied(er) der Patentfamilie | Datum der Veröffentlichung |
|---|---|---|---|
| WO 2014170223 A1 | 23-10-2014 | BR 112015025959 A2 | 25-07-2017 |
| | | CN 105189444 A | 23-12-2015 |
| | | CN 108164417 A | 15-06-2018 |
| | | EP 2986589 A1 | 24-02-2016 |
| | | ES 2673104 T3 | 19-06-2018 |
| | | HK 1250509 A1 | 21-12-2018 |
| | | JP 6501754 B2 | 17-04-2019 |
| | | JP 2016515645 A | 30-05-2016 |
| | | KR 20150143785 A | 23-12-2015 |
| | | RU 2015149480 A | 24-05-2017 |
| | | SG 11201508639W A | 27-11-2015 |
| | | TW 201509895 A | 16-03-2015 |
| | | US 2016068464 A1 | 10-03-2016 |
| | | US 2018050977 A1 | 22-02-2018 |
| | | US 2019077742 A1 | 14-03-2019 |
| | | US 2019112255 A1 | 18-04-2019 |
| | | WO 2014170223 A1 | 23-10-2014 |
| EP 0194620 A2 | 17-09-1986 | DE 3508702 A1 | 18-09-1986 |
| | | EP 0194620 A2 | 17-09-1986 |
| | | JP S61212531 A | 20-09-1986 |
| EP 3450422 A1 | 06-03-2019 | BR 112020003870 A2 | 08-09-2020 |
| | | CN 111051273 A | 21-04-2020 |
| | | EP 3450422 A1 | 06-03-2019 |
| | | EP 3676241 A1 | 08-07-2020 |
| | | JP 2020531673 A | 05-11-2020 |
| | | KR 20200045524 A | 04-05-2020 |
| | | SG 11202001645P A | 30-03-2020 |
| | | TW 201925157 A | 01-07-2019 |
| | | US 2021032386 A1 | 04-02-2021 |
| | | WO 2019042807 A1 | 07-03-2019 |
| WO 2017046110 A1 | 23-03-2017 | CN 108124444 A | 05-06-2018 |
| | | EP 3350153 A1 | 25-07-2018 |
| | | ES 2738991 T3 | 28-01-2020 |
| | | JP 2018527375 A | 20-09-2018 |
| | | KR 20180053716 A | 23-05-2018 |
| | | RU 2018113514 A | 16-10-2019 |
| | | SG 11201802078S A | 27-04-2018 |
| | | TW 201722900 A | 01-07-2017 |
| | | US 2018251419 A1 | 06-09-2018 |
| | | WO 2017046110 A1 | 23-03-2017 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2014170223 A
- DE 102006015710
- EP 1995232 A
- US 20070010394 A
- GB 2004886 A
- EP 0006248 A
- DE 3508702
- EP 0194620 A
- DE 2143582
- US 2577445 A
- JP 2006028067 B
- US 5929275 A
- WO 2010030339 A1
- WO 2008071508 A1
- EP 982314 B1
- WO 2008012128 A1
- WO 2008006633 A1
- WO 2007036424 A1
- WO 2007028660 A1
- WO 2005090276 A1
- EP 1294668 A
- US 7141702 B
- DE 3213681
- US 4408079 A
- US 2848499 A
- JP H04173757 A
- JP 3069420 B
- EP 0317909 A
- EP 3450422 A


### In der Beschreibung aufgeführte Nicht-Patentliteratur


- Ullmann’s Encyclopedia of Industrial Chemistry. Wiley-VCH Verlag GmbH & Co. KGaA, 2012
- **KRILL ; RÜHLING.** Viele Wege führen zum Methacrylsäuremethylester. WILEY-VCH Verlag GmbH & Co. KGaA
- **R.J. CHANG ; SYED NAQVI.** *IHS Chemical Process Economics Program,* Mai 2014
- **S. NAKAMURA ; H. ICHIHASHI.** apor Phase Catalytic Oxidation of Isobutene to Methacrylic Acid. *Stud. Surf. Sci. Catal.,* 1981, vol. 7, 755-767
- **NAGAI et al.** *Sumitomo Kagaku,* 2004, 1-12
- **E. BILLIG ; D. BRYANT.** Kirk-Othmer Encyclopedia of Chemical Technology. John Wiley & Sons, Inc,
- **R. FRANKE et al.** Applied Hydroformylation. *Chem. Rev.,* 2012, vol. 112, 5675-5732
- **DESHPANDE et al.** Biphasic catalysis for a selective oxo-Mannich tandem synthesis of methacrolein. *Journal of Molecular Catalysis A: Chemical,* 2004, vol. 211, 49-53